# EUROPEAN PATENT SPECIFICATION

(11) **EP 2 420 578 B1**
(45) Date of publication and mention of the grant of the patent: **14.06.2017**
(21) Application number: 09843193.5
(22) Date of filing: 17.04.2009
(51) Int. Cl.: C12Q 1/68

(54) **A METHOD FOR SCREENING CANCER**
KREBS-SCREENING-VERFAHREN
PROCÉDÉ POUR LE DÉPISTAGE DU CANCER

(43) Date of publication of application: 22.02.2012
(73) Proprietor: National Defense Medical Center, Taipei City 114 (CN)
(72) Inventor: Lai, Hung-Cheng, Taipei City (TW)
(74) Representative: Chaillot, Geneviève
(86) International application number: PCT/CN2009/000411
(87) International publication number: WO 2010/118559

(56) References cited:
- CN-A- 101 250 588
- LAI ET AL: "Hypermethylation of two consecutive tumor suppressor genes, BLU and RASSF1A, located at 3p21.3 in cervical neoplasias", GYNECOLOGIC ONCOLOGY, ACADEMIC PRESS, LONDON, GB, vol. 104, no. 3, 14 February 2007 (2007-02-14), pages 629-635, XP005894030, ISSN: 0090-8258, DOI: 10.1016/J.YGYNO.2006.10.003
- HUANG ET AL: "The study of promoter methylation of multiple tumor-related genes in squamous cell carcinoma of the uterine cervix", CELL BIOLOGY INTERNATIONAL, ACADEMIC PRESS, GB, vol. 32, no. 3, 1 March 2008 (2008-03-01), pages S5-S6, XP022551098, ISSN: 1065-6995
- RATHI ASHA ET AL: "Methylation profiles of sporadic ovarian tumors and nonmalignant ovaries from high-risk women", CLINICAL CANCER RESEARCH, THE AMERICAN ASSOCIATION FOR CANCER RESEARCH, US, vol. 8, no. 11, 1 November 2002 (2002-11-01), pages 3324-3331, XP002496213, ISSN: 1078-0432
- YU J ET AL.: "Methylation profiling of twenty four genes and the concordant methylation behaviours of nineteen genes that may contribute to hepatocellular carcinogenesis", CELL RESEARCH, vol. 13, 2003, pages 319-333, XP002681905,
- SAN JOSE-ENERIZ E ET AL.: "Downregulation of DCB1expression in acute lymphoblastic leukaemia is mediated by aberrant methylation of its promoter", BRITISH JOURNAL OF HAEMATOLOGY, vol. 134, 2006, pages 137-144, XP002681906,
- IZUMI HIROYUKI ET AL: "Frequent silencing of DBC1 is by genetic or epigenetic mechanisms in non-small cell lung cancers", HUMAN MOLECULAR GENETICS, OXFORD UNIVERSITY PRESS, SURREY, vol. 14, no. 8, 15 April 2005 (2005-04-15) , pages 997-1007, XP002463138, ISSN: 0964-6906, DOI: 10.1093/HMG/DDI092
- GAO S ET AL.: "Loss of heterozygosity at 9q33 and hypermethylation of the DBCCR1 gene in oral squamous cell carcinoma", BRITISH JOURNAL OF CANCER, vol. 91, 2004, pages 760-764, XP002681907,
- GRONBAEK K ET AL.: 'Frequent hypermethylation of DBC1 in malignant lymphoproliferative neoplasms.' MOD PATHOL. vol. 21, no. 5, 2008, pages 632 - 638
- LAI HC ET AL.: 'Identification of novel DNA methylation markers in cervical cancer.' INT J CANCER. vol. 123, no. 1, 2008, pages 161 - 167
- SU HY ET AL.: 'An epigenetic panel for screening and prognostic prediction of ovarian cancer.' INT J CANCER. JAN. vol. 124, no. 2, 2009, pages 387 - 393

### SEQUENCE LISTING

<110> Hung-Cheng Lai
<120> A method for screening cancer
<130>
<140>
   <141>
<160> 24
<210> 1
   <211> 5000
   <212> DNA
   <213> Homo sapiens
<400> 1
   ggtcttcacc tgaagttgcc tgactacgtt gtctcagatt gatttcagaa ctgcattatc     60
   agcagggcag ctgttgccta ctaacagtca tggaagtaga tctcagctgg gggatgatgg    120
   ggaaacaaag agaaagatag ggacgaataa aggaaggaat ggggcacaga cagaaagaga    180
   gagagagaaa agaaagaaag gaggagaggc aagagatagt gacctggggt ttcataccgc    240
   ggtgggtggg agaaggagtg tgtgtggtgg gcgtatttct tcgtggtcag aatcaaagtc    300
   agaactgacc tggggaacct gtgttcaaga cctgaacctg gaccagaagc caagggaaag    360
   agaaagttgc ccaagaaaaa agggagaaat ttccaccagg cgaaaataga aatcgctgac    420
   tgggtttgtg gctggagagc tgtccgtggt gctgactcct cctcattggg attccagtga    480
   aggctgagaa gctctgcact ggctgctcct cctctccctt tcctctcccc tcagtcctga    540
   ccttctgaga cccaggatca ttctggctag ctcgatgtct tctcctctct ttctcccttt    600
   ctctttgtcg attaaataat ttttcccgag ttcccagttc atcactcaaa catttctctt    660
   ccattttgtt tcaagactcc tcttcctcca attagttcac tggctcctag attgttctag    720
   gcctccctgg tggtcacatc tctctcttat catcctttga attaaaaaac aaacaaaaaa    780
   catacaatgg caatcataaa agcagacaac atgcactgag cactcactag gtgtcaggga    840
   gcagtctaag tgcctttgca tacattaact catgagcagt ctaagtgcct ttgcatatat    900
   taactcatta caattccaca tcaaccctat tagatgggta gatatattat tattctatta    960
   aacagacaag gaattgaggc ttaaggggta agtgatttca ccaagaccag acagctacag   1020
   gactccaacc tctctggagc ctgcacttct actgaatctg gctcttttac atattccaac   1080
   accttttcct atgacttcct gcccctccat ctggtagcct taatgctttc tattcttgcc   1140
   taatcttcag gggactaggg atctgcattg ctgatttggt ttaaatcaga agggaagcaa   1200
   agtaaacaaa cataccaaat gtgcgctgtg tgggaattat catacgaggg ctttattttc   1260
   tgcttcagga agaggcccta tgttagcagc cccagcctgc attcaggctg attgcagagt   1320
   attttgcttt ttattttcat gtcttagtcc ctgtaccctc gccccttccc cgcctctggt   1380
   ggtctccaga gaacttcgtg tcccctcagc ttctccctcc tacatcctgc ctacgtagag   1440
   aagctcttgc ttcattctgg gaggttacgt gggctctcgc ctacacaccg agagaaacaa   1500
   acagtgtcaa acactcacag agagacgcgc agacacaaac ggacccacac gggcaactcc   1560
   cgagacaaaa cccacactcg atggatccac gcggccgtgg aaacacctgc cgccccagaa   1620
   acactcaggt actcgcgaca cacacagtac agtcacgctt aagggcacca ggattccggg   1680
   tttgcgcgta tgcgcggtcc ctttggatgc tcgtgcgcat agacacaaca ccctacacgc   1740
   cccagaccca cgaaactccc tacggctcag ccccagccca cccgggccgc ccttccctcg   1800
   aggcggcctc ccgtctctcc tcctctcgct tctcctcctc ctccgcctaa agatgtacaa   1860
   aacactcctc ggaagcaacc ccggcgttca gctcctccct ccccgccccc cggccgccgc   1920
   tcccccattc attttcggcc gtcgccggct aagtccctcc cccggcgtag cccggcctcc   1980
   gccgctcccc gcccggagac cgcggcgcac ttggacttcc ctctccattc gccagccgcc   2040
   tcgctcccgg accccacggc tgcaaactga tctggcgcgc ggggaggagg agagcgcagg   2100
   cgagcgaacc cgcgagagag ggagagagcg agcgagcaac agcgagagcg agagcgagag   2160
   agccgggagg cagagggagt agtgaccgcc ttccggagcc gggattcatg cctgtcctcg   2220
   ggaccagcga aggggacttt acggctgagt atgagccagg ctgctaggag ccaggtaccc   2280
   ccacgcctgc agtccccgcg ccgtgcccgg aatgcgagct gcacgcaggg ctctcccaag   2340
   ttcccaccga gccgaataaa aagcgtcctc ccgcagctct ccgccaaaga cggacattga   2400
   ctccaggtaa ggcggcgccg ggtgcagcgc cccgcagccc cgctgccctt ggacccggcc   2460
   ccgggccgca ttcggggcgt ccccgcgctc ctctgcccct ccccctaccg gcacccttgt   2520
   ccgctcttca cctggccgcc ccgccgcctc caagtcttct ccagttctag ggagggggtt   2580
   cctgtgcctg gggctcaaag ggctaattgc gggtttgagt gagtggcgtg tgtgtccccg   2640
   cgcgctcccg acgtgtgcac catggtggga acttgatgtg gtgctagtgt gtttgcgtgt   2700
   gcggcgtcgt ttgcgtttga tgcgtgtgtt cgtggtgtgt gtgtgtgtcc gtgtgtgtaa   2760
   gggaggggtg aagagagaga ggtcctataa cctacttacg gcgcgatgtg tgtgtgcatg   2820
   tttgtacgta tgtgtttgta tgtgtgccct cgtgtgtctt tttaattagg tctctccagc   2880
   ttacacggaa tgggaccctt actataggat cacgtagtca ccgggaaacc cgctgtggac      2940
   ttcctcttgg ggctctgggc ttggggtttg gggaggatta tggggctgta gatggcacct      3000
   tatttagccc aatgttggta cgcttgaagg aaaattcctc caaacggtgg aatcctgcta      3060
   cactgggacc cacagcttaa tatgaaagag acatggccaa cccccgaggc aaatgagacg      3120
   ctgtcacttt aaattctaca ctgggcagac tccaagattc tgatgggaat ttggagacac      3180
   tggatagtgg gtgacagaga aagggggaag tcagcggtgg gctccttatc tggggcttgg      3240
   aaagtctggg atagggattt accctgcatc cccgtttgca atcaacagag cccctccggc      3300
   ttctgttggt tttggggagg atctggcaag tttgcatgga tccccctcag gggaaaggag      3360
   aaagcgtcct cggggacttg ctcatccatc acagttgcaa agggtctcag aggaaatttc      3420
   atctggggcg gctgtggatg atatggaagg agatggatgg ggcactttcc taagacagat      3480
   tcgtctttct ttccccattt caggcgggga agcccccaga gagtctctcc ctaaatatgc      3540
   ctctccatgg ctcccctgga gttaggggga tattgagaga aggcagagag gtggagaggg      3600
   agagagagag agcaagagcg agagagaggg agacagagag agagagtgtt tcagtactga      3660
   gggagatcta caatttgaaa aggggctgtg agtgtggaac ccatgacaga atgtggcagt      3720
   aattgactta aactgctgtc ggtttgcacc tcgcgtctct cacttggctc caaaatactg      3780
   ccaagggcag gggggcggtg gaggaatctc agccaggaag gtagtttggg tcaaggccct      3840
   ctttcttctt tcctccatct cctgggaggc tctttaggta ggtcccttgg ccaccctggc      3900
   ttggtactgt tgggcttggg ctctggggcc agccattgat ttgattccca gctgcctctt      3960
   aaaggcttgc cctactcagc aaaaatgctg agttttactg ctgttagcat ggcttccaga      4020
   ctcggcagct gttactttct caaggtaagc ggcagccgtt ctgctctcag gcagggaggc      4080
   taggagaaga caagggctgt gacgctggga cacagcctct tgtctagctc agcacggagg      4140
   ggccgctgaa aagccctctt aggggaaaaa agtagaaata tatttggcct aaaaaatgta      4200
   cacatatatt ctaggaagat atatatatat atatatatat caatcacaca cacacacaca      4260
   cacacacatg tgttcatatt ctggggagag ataaaagcaa acatgtattt gtaggaacct      4320
   gccctagtgg gtgggttcta cctgcaggca cttgcagaca taccattctc tgtccagagg      4380
   ctgatacctc aggctgaagc cttcacatag ccacagagaa tctccttcag gaaactcatg      4440
   tcagggcaga gctccctgga ttatcttggg tagggggtac tagggccaag gaggagaccc       4500
   atttttggga actgcctcat tttctctctt tcctaattca cagacgatcg aaactggaag      4560
   gaatattaaa gagcagaaag gcaggactct gtggcctaaa gagggaatga gaattcccca      4620
   acattcagta gtgggttagt ggcatgccca ggactagaat ctaggcttat caatcagcta      4680
   gatcaatacc ctttccactc ctcccagcaa tatacactct aggactgtat attgctccca      4740
   cccctatctt gccaacctgc caccatgacc cctaccaaga caatcgcttg tcttccctca      4800
   tcaaacttca cattttcaga gatgcatttc agggtttttc tatctggaaa catggccctg      4860
   aagagaaaag atccaatgcc tcaatcacta ttcccgaaag acacacacac acacacacac      4920
   acacacacac acacacacac acacacacac acacacacgg tggtgggggg aggactgaga      4980
   gagagacaga gagagaacat                                                  5000
<210> 2
   <211> 5000
   <212> DNA
   <213> Homo sapiens
<400> 2
   agaacaactc tgaagcatca tcccaccttc agaatcctcc ctgcagggat gcctgaggct 60
   tggtcaattt ctccctctgc tcaagcttgc ctccctccat tccctccctt tatttcctcc 120
   cacaaatgtt gactacaaga gcacttccta aaaagcctcc ctctgcctgc taaacaccat 180
   ctcagaatct gcttctagga ggacccaacc tgtgcagaaa ttttatgagc aatttgcctg 240
   tccattaaaa aatacagcat agactttttc caggttagct ggagatggag tttttttgta 300
   tcaaaaaaag agagctaaac tgagaagaga agcaaaggta gaagaataag gttagactct 360
   ccctgtataa ctgaatttgg gattgggaat gatatcttgc ttctctatga aatttagaaa 420
   ctgtcttttg aagtcttcat ccagtcacat ccaagagtgg tccaggaaga aaaaggaaaa 480
   taagaatgaa aaaaaaacaa aaaacaaacc ttaaaattgg atacttcttt gtgccttgca 540
   ttaagcctct atgttactgc ccttgtccgt ataagcaact ctagataaaa tcctttctta 600
   gaccatctaa tttatctccc cgtgccattg tgaaaatcgg gcaggaatta ctgcgggaac 660
   cagtgactga atgggcagtg gtgtctcgca gctgtaactc agagtttaca agggaaggat 720
   gaacagcagc catccttcta aaggcattct tcctctcctc cagcacagct cagtctgtgc 780
   atgcctcacc aaggagaatg agggaaggcc ttgttgactg cccagcccag agtgtgagga 840
   acagcagttg acatgggcgg ctggacggga tagaaaccag catgaacatt tagtgacacc 900
   taaggaagag gtgaaaatgg gggagaaagc caagaaccat cagacagctt ctttctgttc 960
   ttgccttgga tcttagggga acgtatgtgt tcaagggtgc cttcctccgc agggcctccc 1020
   atcccttggc acaaacttcc aaacgatgag tagacatgga atgggcttct tctgaagact 1080
   gggtcaccat ccaactcagc cttcggagta ggcggtgggg ctgggggaag aggacggaac 1140
   catgaggtct ctccatcact tcccgggtgt gttgagaggc aagcaaatgc ggtgggtggg 1200
   cctgggctgt agggcccctg ccatttcggg aggggcctcc tggtgtttag ctctgggatg 1260
   tgtgaaaatg tgttggtgaa aagcgagagg cttacacagc ccttcagggg aagaggggct 1320
   ggggcgctgg gggcggcgtc gggatgagtg cagaagagac gaggcctctg gacagcggag 1380
   gaggagggga gggcgccgag gcgcggtgcc agctgccgcg cacaggggcc ccgcggcgga 1440
   gccgagccgc gggcacgctc tgccctgtcg ggagagctcc gggagcggcg ggaggggcgg 1500
   aggggcgcag tggggcccgg gcggctgcgg ccgcggagcc ggggcacctg aggaggaagg 1560
   agggtgggag cgagggaggg aggggacggg cgcagaccga aagtggggaa agaaggtgca 1620
   ggcaggcggg caggcgggcg ggcgccctgg cccagggccg cgggtgcggg agcccggcga 1680
   ggtcgagctg ggcggcggcg ggggccgcgc cgagggagga ggggaaggcg gaggcgcggg 1740
   gagcgtgttt ggggcgccgc ggcggggagg gtggcggccg ctggtgcgcg cggggcgctg 1800
   tgtatgcgcg ctcccccgct cggggaggaa gatggcccaa aagggaaagt tggggtgacg 1860
   cgcgcggtcc ccggaggctc ggcggggggc accgcggcca gcccgacgga gcggcggaca 1920
   cacaggccgg ggggcgcgca gtccgggcgc cgccgcggcc gccccctcac tgcaggtggc 1980
   agcgggtgcg ctgggtcccg gcggccgcgg gcgcgggcgg gcgcgcgggg gagcccggcc 2040
   gagggatggg ctgcgccccc agcatccatg tctcgcagag cggcgtgatc tactgccggg 2100
   actcggacga gtccagctcg ccccgccaga ccaccagcgt gtcgcagggc ccggcggcac 2160
   ccctgcccgg cctcttcgtc cagaccgacg ccgccgacgc catccccccg agccgcgcgt 2220
   cgggaccccc cagcgtagcc cgcgtccgca gggcccgcac cgagctgggc agcggtagca 2280
   gcgcgggttc cgcagccccc gccgcgacca ccagcagggg ccggaggcgc cactgctgca 2340
   gcagcgccga ggccgagact cagacctgct acaccagcgt gaaggtaaat gccccgcgct 2400
   ggcacacgcc gtgggggccg tccgccccgt cggcggggct cgcacgggta gggggctccg 2460
   gcggagttgg gtgaccgtga ggcggttggt ttggagaggt tgtcactaag gaggagttta 2520
   cttttcattt gtggagatga tgggagccca ggaaatgtgg tcagaaaaag gcccctggag 2580
   gggtcctgga agcgtcctta gctggtcctg ggggactggg cggggaaggg agcgcagaag 2640
   gaagcaggtg ggctggcctg ttcctccttg agggcaggaa ggctgtggct tggtttatgc 2700
   aggaagaggg gtggggacca ttgagagcat tcggtggcca gtcctgttga atgaaatctg 2760
   agcactgagc tggatttgcg tgccttgtag gtgactggtg cagttgcagc accaggatag 2820
   atagtgcccc atattccgat ttttacctgg gattaccagc caggctggag tctcagcaca 2880
   ggaaccgagc gtagggattt gtgaatgaat gagtgttcgt gttttaagag atgtgggaac 2940
   ggagcagagt ggaacctgtt gtttgtcact gtaacgtttc tctgggttgg ctgcatccta 3000
   gacagaattg agagaaccgg gccatgagtt cggagtgtca gcagagccac cgtgagggga 3060
   cgtggtttcc agtgcagtac agctgtctga ggatgattct gcacatacaa ctgatcttcc 3120
   cagagagtgg gattttgagg aagtggaaaa aattgtttag atggttaaag cagtcgctaa 3180
   atatttattt cttaaagaca gaagaaaaat aattatttaa atagtgtcct cccgtatgtt 3240
   ctcaaagtac cgttaaatcc aagaggcttt tcattgtgta aatctgggca ctgggtcttt 3300
   tttcctttca gcaaacaaga taacaatggc atatcctatt gtacagagag aaaaaaatac 3360
   tcctaatgtc agatagaatc acagccttta cctggtcaat aggttaccaa gaaccattcc 3420
   atggattatt tgtcaaagac acatttgtat ttttaatagt taaaaacttg gtcttcattg 3480
   gatgaaggca gcccacagtg gaggagtgag gagaaggata acatgttttg gcttcaatct 3540
   ggaagtccaa aagtttttta atgaacctat ttttttttta acagcatctg attgtttaac 3600
   atgaagcttg actgatgttt gcttgtggtt gcagtgtttc tctggcagta atcataatca 3660
   ccattatagt aaaaacatcg tttattgagc acctactatg tgccaggagt aagctttctc 3720
   tcctaactat tgaatgttaa cagtgtgatc ttagcatagc atgtctgaag actagagtct 3780
   aggatttatg acagtacaaa cctaccagtt gcccattcgt tacagaggca tgtgctgaac 3840
   gctatacttt gcttttgtca agctctctct ctctccccct ccctgttcac acaaagttag 3900
   ctttaggagc aattttcagg gatgaaaatg ttaaatttgg tgaaaatatt aagttgggat 3960
   attatcagta aagaccatcc ttttgcctga cggttgaaga cctgaagcag tagttcaaaa 4020
   ttgtgacagc tcatgaactt tgtgatactt tttttggttg ctccagagaa attgtgaatt 4080
   tttgttttct agaatatcta taatctcagt ttagtacttg gcaactctag ctactccatg 4140
   tttgttcagt agaagaaaga atactgaatg aacccattct gtacattttt aatacttccc 4200
   tcaagaggct gcttagagac caaatgaagt aaaaactgaa gggacaatga gccccaatta 4260
   ttagcagctt gtataaactt gtattaatct cattgctact tggcttaagt attaaaaggc 4320
   ttgacctggg taggagttta actcattttg ctttatcaag aagattccca aataagagct 4380
   taattcactc taacccatta ctgggaatgt tggaaatgaa acccctggtt cattgactta 4440
   acactacttt gcattgtgtg gatctcagaa agtatatatt tgaagtgaaa tttgagacca 4500
   gagttattta tgcaaatctt acagaattat tatcacagaa ctgccctaat atatttaata 4560
   gtatcctttt aggcctgtga ctcggtgttg tattgtcttt tctcaatcgt aaagttcaca 4620
   tgttctgtgc ccttttgttg ctgagataat tttataaaaa ctttacattc atttttttgt 4680
   agactaagca ttcttttttt gccgtaagag tttgagatag ttgctaggca cgtggtggtg 4740
   ccccacccct ttccaaagca cacaggtgga aaaataaaat ctttaccatt tggtcctgtt 4800
   tacagtggtg gaaggttgta atagtttcca ccccctcccc ctggccattt tacatcaatt 4860
   atgaagtgta tcctcattat ctacttggcc tctggtattc cccagagtgc aaaaatgtgt 4920
   gcttgatctc tctctctctc ttttctagca ataattcatc tcattaagct tttagaatga 4980
   tgaggcatta tgatgtcaaa 5000
<210> 3
   <211> 5000
   <212> DNA
   <213> Homo sapiens
<400> 3
   aagaaaaata gacctattgt aagtgggata caattatttt tccctaacct ccagccctgc 60
   agcatttcta gtaacagatg ttagatgaga agagttattc ttctaagctt tacagaatgc 120
   taaataaatt atctcaaagg ttgactcagt aagaatttca aagagttaat tgtcagtatt 180
   gaactttgtc aactatcatt ttcatttatt catttttgag tatctatttt gcatgaggca 240
   ttgctctagg gagttgggac atatcaataa agtcatcaaa gttcttcaac attggagctc 300
   aaattctgtt ttgcgggggg gagtagggaa atagatacta aacaatagat tttataagta 360
   attattttgg tttgttagaa agtgatacgg gaaaagcaaa ctggaggaca gtgaatcagg 420
   aataggagaa gaggttgcag cttagagtgg tttgggatga aatgatagtg ccacagaaat 480
   ggagtgtgcc acacgcatgg gggagtaggg ggtggagcac gttgctagag aggggctaga 540
   catggagata tgtctctatt ttcagcagag aaggcctgtc aagatattaa aagtacatct 600
   cattgtcccc ttctcccact tgttggagtg cttcctggcc ttgtctctca gagctgaaga 660
   tgacataaat cataaaagaa ataaatggta ccgcctgggc tctctgatct tataaggtcc 720
   cacctaattt aagctctggt ggatagccta tttcccatta gttgtctcaa ttcacagagg 780
   atggagaaag aaagcactga atgagatgtg cagctcaact caatgggcac ttgttagccc 840
   caattatttg gctaggacaa ctttattcaa gttaatggaa tacaaaactc agcagttctt 900
   atggagactt caatgtgctg tagaacaaac cacagataaa tgaactgtct gaaagaataa 960
   tatgaggcac ggctaaacaa gtgttgtgga agagaaagat cactgtaatc tagagttagt 1020
   tagagttagg gctttctgga aaagtgagac caacattaca ccttcgaaaa ctagtgtggt 1080
   ggtggtgcaa gtggaggaaa atgggaaaga ggcaacagtg tccataaaga cacagaggca 1140
   gaaagggcag aggccctggg atagttaaaa gagaagtcta gctgagggac ttcttgactt 1200
   gactagcagc aattagccat gactaataag gctttccaca ctccaaagac ttagatggag 1260
   ggataaaaaa ccatctaatg gcagactgtg gtagcctccc tagagacaca gagctgggcc 1320
   ggatgagtcc aggcactgac gtgatccatt atctttcacc ttaaagagta aaagggaaac 1380
   taaagttaat tacctccacg aaacaaaaag gtgccttctt gtgcttcaat tacatggata 1440
   tattctacta gtctaaaagt atcttctcac ttctttctgt cactgtgagg acttgagtca 1500
   gaagaaagtt taaatacagt cattgagctg gaaagagtgg aaagagaagc aaagaggggg 1560
   aagctgtagg aaggacgaag tcacccccaa gatacatggt tactgcttac accaagcaag 1620
   ctgccttggg aacgcttccc ccgagcagcc agaatgctca gcagtggaag acacctctat 1680
   tcctgtaggc gagtcctggg aagctggtca atctgcaaat gccaattccc agcagtgagc 1740
   tcggtccacg tgtaaatcaa gatttgggga aagagtaggg tgggtggcat ggttgacaat 1800
   gtcatcagct ccctcctctg actcctgtgg tcgtgccccc atctactctc actcagctac 1860
   accccacctt cggatttgtg atggacgctg ggtccctagt aaccacagca agtgtctccc 1920
   ccgcacttcc cccttcccca cccccacccc cacccccaac caccacccca gcgatggagc 1980
   ctactctgct ccaagccgcc gctaagaccc ggagaagcgg aatttcactt tgaaattccc 2040
   ttgcctcgtg agggccggcg ctgggcatgc tcagtagccg cggcgctgct gctgggctgc 2100
   tgggctggcg cggagtccac cctgccgtct ccgccttggc ttctgggcgt ccagaaggcc 2160
   aggcatttgc cgcctctgag cgcttctgtt ccccttaccc gcaacctcct actgctcttc 2220
   ctctctccct ctcttaggga ggttgaagct ggtgctggtt tctgtcggcg ccacagactg 2280
   actgctctgc aaaccccagc cgaggacctg aatcccggag actagaagac ccttggcggt 2340
   ggctctttct aatagcactt tacctgaagt ggggtcgtgg tggagtttct cctccacctc 2400
   tcaatgcaaa cactatgcgg agagcagtct gcttccctgc gctgtgcctg ctccttaatc 2460
   ttcacgctgc aggtaagggg ttgccaggct tagagccgga gctgtgcatg agatgggaaa 2520
   ctgcacatgc ttaaggactc taggaaaagc ttgccttgcg aagaaagcct ttctaaaaag 2580
   gtaaaacagg acagtacctg acagggaagg ggtgagggag tcgtgcacct gttggaaaac 2640
   tgaggccgaa aacttaacct aaaattagct cttgattttt ctttacttta tacgaaaata 2700
   gtgaacattt tcaatatgaa tacaggtttg ctcccccctt cgcctcccct ccgtctagat 2760
   tccctgctct tgtttctagg ctatgcactt aactgtcaat ttttcaggag agggataaga 2820
   catcctgcta gatgtaacct tttctactgc agcggctact acattcataa ggtctcttgg 2880
   tctagcgagc gctcatagga aggcattggc tgtaacctga tggaccacat ctccgcccaa 2940
   aagatcgaaa tacatggttc acattagtga tgctgcacca ggctcctttg gcccctgctc 3000
   ttgtcactcg aattttctaa gccaataaga ggaagaaaag gtttcaaata gaatcctctt 3060
   gtctctttca gcaccgtggc tagcgtccgc tggcatatta aaaaaaaaaa aaaaaaaaaa 3120
   aaaaaaaaaa acaattacag ttccaaagct gacaaccctg ggttaggcac tgtccctcgg 3180
   acagatttga taaggtgtaa ccaagagtaa cagtgagact gctcccaaat ataaatagat 3240
   ttgaggcacc agaatcaata tcaagtctcg tgaggaagca aaccttggga agtacctgag 3300
   agatattata tgtggtgtcc ctgctttttg catacatagc acctctattt aatgcagaca 3360
   gacttgctag gctgaattcc ttatcattgt cacacacaca tgaaccaaaa taaaatatgg 3420
   cagggagatt ttagaaaccc tactgtgaca gcagttgtcc atgcagtagt cctgtaacaa 3480
   ccagagacga ggcattttcc ctgagtgtgc ttctcatcca atcacacata tccattcatt 3540
   ccttcaagca acatttgctg agtgtatact atgttctagg ggtaatgagt aaaaataatt      3600
   gttctgaaat tacaatcctt gcagtatctt ttttaaaaga gtgggtgaat tttattctct      3660
   gtgtacaata gtaggagtga aatatggtac tttcttattc cagctaacgc tattatttaa      3720
   atgtatcatg aatcttttga gcaaataatg cataggatta ttaaagctat tattaaatac      3780
   acatgttaat tgttataatt atgatgttat attgtggagt tttcaatagt tctttggcac      3840
   attttgagaa gtagaaaata gctatgactt gattattaaa ttatttgcaa acagctgtag      3900
   tgaattattt cgttaccaca tacaattctt caatgctgaa atgtgggatg aaaacataca      3960
   cattgggttg agttttattt atagtattgc ttaataatag actgacacaa acattcatta      4020
   cagaaaaatc tcaattctta ctaaggtagc aatgatttag ctggtctgcc aacaatatca      4080
   gtgtagtcat tttttcaaaa ctaacatata aaaaagttta aagatcacat ttgatacatc      4140
   ttgagtaatt atatcttaaa agttataaat aaactgaagc atgtgaaaaa catgtcttga      4200
   tttactgtag atgaatattc gggatgagaa aaatgaggac ttttgctcat cagatgagaa      4260
   acctagaagc ctgtagccct agattttacc tggagttctg ccaaagggta tattaccaaa      4320
   gccaatccaa tccatctcta cttcaacttt ttaatttgca aaacaaagat aaaaagtaag      4380
   attaatcaac caattttaat ttaataagaa aatgcattta aggacatttt tatatttcaa      4440
   ttgcaagata ctattcattg tattcatatt taatatagta aaatacattg gtgttttatc      4500
   acagcagatt gtaagtttaa tattatagtt ctcttgccaa tttcatttta aatgcatttt      4560
   aattttaaac agcaatttgt gggtcacaag ctgtcatcag attaaaattt atcacagtta      4620
   tctaataaca ttgttagggc ttttcatcag aactaaaaca aacacagctc cttggtctaa      4680
   gcttctatac atttggagat taagaaaata agtggatggg atgacaaaga tcaagaaatc      4740
   aagatattaa tctctacttg ttgaaggtgt gagtttgtta cttttatgga agacttttct      4800
   ctcagaacag aggctgactt cataaagaag ggaaatgaat tctatacatt caggcctctt      4860
   taactgtaaa gctaattgat ccattttggt tacgcccagg gtatcagcaa ctgctttatg      4920
   ctggtggaac tgatgacacc ttataatgct ttagagatgg gaggaatgtg ctaacgggat      4980
   gtaagtgttc tttttagcta                                                  5000
<210> 4
   <211> 5000
   <212> DNA
   <213> Homo sapiens
<400> 4
   gacgaccctg aatgatccct gcctcctagt acttatactc ttgtgtagcc tccacctact      60
   tagactcaca gctgattttg tgaccaagag tataatggtg tatgacttca gaggcttggt     120
   tcagagtcag aagtggccat ttcatcatcc tctaaatttc tctttggcag attaatgaat     180
   gtgagaaagt aaaaatggta tagaagaaag ttagcctgag agatggctgg ctttgtacct     240
   gtttggtctt tttccttcct ttcctccata agcaaatact gacgcatgta catatagaca     300
   cactcaacac agtgggatgt taaagggaaa ctagagccac tcgccccttc ccaagatcag     360
   agaacatagg aaagatgatg actttttgca tctaccctct cctcttgaac tcgcaacact     420
   gtcttgaacc acaaataagg tcaagtagga taaatctaca actgtctcct caactgtccg     480
   ctcacaagtg tgaaattcaa ccatccttgc tgaagttgtc ttttaatatc agtaatcagc     540
   aataaccaac ctctgagctt tgcttatatt attaaatcat tttatttgac cctcaggaca     600
   aagctgcaag cccagcatta cattcttcct tttatggaca aaaaaaaaaa aacaaaacaa     660
   aaccgagata ggcacgaaga aactaataaa tggctgaaat gctatttgaa tccacatctg     720
   tctaattcta aaagtgtgct ttccaccaca tcaccgcacc tacaaggaaa atgcagcctc     780
   taccctctcc tgaagaatgt gtaaagaggc aatatggtgt gttttggaaa aagcatcttt     840
   gcagaaagaa agcattagct tcagtcatat tagcatcctt atccagcagc agtagcatta     900
   ttttacatat cagttactcc tccccaacaa ctcaatgaat tagatgccac aatcatcctc     960
   actttcaaat gtggagaccg aggcacatct ctaagactgg agttcaggca ttctggctcc    1020
   agtcttagag atggttaagg gttcacactc ttaaccattt attacaccat agagctcacc    1080
   aggtttgagg gaaacaggat caaatcaaaa gagtcactca ggactccagt cctcactcaa    1140
   ggacaaactg ttccacctcg gacagggaga gtttccgcat tctgagaccc agcataacag    1200
   gtcctgaccg gcatctggca ctcggactcc caatcatact ggatcacact ggctcgggat    1260
   gtgtaaagtc cagggcttct cacatttgat gacaccaaag ccgcctaaaa acaagagaga    1320
   attaacaact acctacggcg gtctgatatt tgcccaagag atgccgcccc ataaaactcc    1380
   tttacatctt tataacgttt ttattttgcg ttctccttca taacccacat ttaactcacc    1440
   atagatgtaa tgtttaaaat tagttaccag ataaactctt acgcttccaa actttaaggt    1500
   tccttcgaaa ccttctggta aaactgttgt tccacggaaa tgggaacgta acggatgagg    1560
   caatcttcca cagccgcaca cagttgtgta tccaccgcta aacggtccca gtcatacatt    1620
   caacgaccca cgcggagtca gaagctacca ccacacactg tcaaaatcac gcacacacag    1680
   tgacggcccc ttgcccactc ggtcactcgc ccacaatctc tcgctagaga atcacacgca    1740
   gatagcacac ccagcaccac agaccccagg aagcaaccca gggactcgaa cacacgaaca    1800
   gcactcctcc gcgcactgcg caggcacgcc tgcgtccggc tcaccctgaa acatcgcgag    1860
   atccggcttc aaggccgggc tgctgccttt acgcctaaag actatgtttc ccggaagaca    1920
   ctgcggcgcc ggccctatca tggcgcagca tcggtgtgct ttgtgcgtct gcgccatctt      1980
   ccggctgcgc acggcgaatc caccggtacc gtggtggaag cgcgccctgg gctgccgggg      2040
   gcgcggccgc ggtggcactt ggacccgagg aggcggcagg tgagaggttc cggagctttc      2100
   caggcgctct ggggtccagc aggagctggt gcccgggccg gttgggtctc aggcctgaga      2160
   agaacgcaga cgtctcgcct catcgtcgct ctgtggcttt accggcgtga gactacattt      2220
   cccgccggcc ctcgcggcgt gcgctttctg gcgccccctt tctgcttcca gcctcatagc     2280
   ccaggtgcat ggacccctta ggtgggtgcg caggggtctc cgaccccctg aaattgcgga     2340
   tgttttgcat tcactgttat gcgtgccttt tttttttttt aatctgagag gaaatcttgt      2400
   ttcatgaggt tctcagagag ttacaagacc ccagaagact tagaaccgct agtttagaaa      2460
   aacttatact tgggaacatt ttatatgttc aaattctatg tccagcaatg gggaaacact      2520
   tgagtggatc acaggccatc ctttgtaaat aggatatcgt gcagttaata caaataatgc      2580
   ttataaaagt ggtcataata aggaaaagct gcctattaca tcatattaag caacattcat      2640
   aattatttca cttacacgtt acttagaacc aaatgtttgt aatgcagtag gcaccttata      2700
   tactccttta cagagtactt cttgtttaac agtgagctca agaactggtt aaatgacaac      2760
   actgcgaata cttgggtgtt ttttcttcca atttttttgt tttgttttgt ttttagcctg      2820
   tgtgttagtg agtttgactt ctttttgctg aatgttctca aaggattcag tagttaatat      2880
   ctttgcttgt cagtttttga tagtcatttt tgatagtcac ttggcatccc attatatggc      2940
   tgtgccaaaa atgattcaac ctcttggcta cttgtggact tgcctactta cgtactaatt      3000
   ttcagttctc ctatttagca aatattattg gacatgcagc tttgtgcact gctgtgtctc      3060
   tatggaagga cacatttatg tgctgataat ggagcactgg gcgtgctgtg tgtaatttgt      3120
   ctcttactgt tccttccaac tctatcccat ttgttgatgc aaggcatgct ccatgaagtg      3180
   gcatgcacaa agcctcagtt tgggctgtag agctttttgt atatttggcg gttgggaaaa      3240
   gagccaggta gactttcaaa ggtgttatat ttgctgcagg agagagttaa atggcctagt      3300
   cctggaaagg ctttattcta ggctggggct atgcaaatga tgcaggcctt ctatgctccc      3360
   tggcttgagg gaagccctcc ttgtccttga ggtaacatgt gggtctcagt cactagaaca      3420
   gccctcagca tccctgtctt agacttgcat ttgcagcaaa acagaaccac tggatgttta      3480
   ggggaaatgg gagggaaagg accactggtt cccctttgcc ttgcagcagc taaagctggt      3540
   ttcctggggt tgggtgagga tccctttgcc tccccctcct tctcattttc ttggtgggca      3600
   gagggcaagc aggtggggac ttgtaacttg gcattctagg aactgtgagg caagcctagc      3660
   ctctcatgtt tgctctttcc tcccccagct ctaccgtcgc aggactctgc ccttccccaa      3720
   gagaggaacc agaaggagga cctatcagcc cttgaaattc taaaagtcat gtcccttgtg      3780
   agtttttaaa tcccataaat atttgctttc cttatcttga aactttccac ctgggttcca      3840
   tccagaaatt tggttctcag actttcccat tttagggaat aaaaggacca ggcaggagtc      3900
   tcctctccca ttgtcctctc ccctctgaca aatgcccaca gatttattct ctatagcatt      3960
   ctgcatctcc tgatggtttt cgtctgctag gaggagaaac tcatttccct gtatgaaatg      4020
   agagcatcct ttgtaagaac tgaagtttga agagatggaa tgaatgctta ggagcaatca      4080
   catatagaaa agcaggggag gtggtgttgg ttggtttcag tttcagcgca tggtacagtg      4140
   tcctgctttg ggaatgatta atgattggat atgtaattga ggtttggctg ggaatccatc      4200
   aaaaatttga tgccagaatt aaagtctagt ttgacgttgg acagagcatt tgaatctggg      4260
   acatcctcca ttagtttctt tagcttaaga ggaagaaaag tacagagctg aggatggtga      4320
   gcgatgtagc aagtgagatg atcatctagg tgggtgagtg gtcagggctt ggaaaagcct      4380
   tagagatctg atccaacctc gggatccaaa ataagaggat gtttgagtca gttgtttcac      4440
   ttgatagaaa attgtgcact caataaaagt tatctccatg gtttttccca tgaagaaaaa      4500
   tgcttgtgtt atgttccaac ataatgatga actttcatct ccaagaaatt cttgacttac      4560
   atgaaaatcc tgtatctaag atagaaaaca tgtttccata attttaaaaa ataaaatatt      4620
   tggggaaaat ccatcagaaa tattttttgt tgttattgtt attccaaaag atagaagtga      4680
   taggtctcac cttgagaatt tattgtatgc caagtataga gtgggcagtg tgctttatta      4740
   cactgtttct taacaataag gaaaactcca tttagcttga ttttaatttt catcccagac      4800
   ctgtacacag agcattgatt tgcagttaaa aggaatgttt gagaacaatt tgatcattct      4860
   gttttactca tccccatttc ttctgtcacc tttcacattc agtcccaccc ttcttgttca      4920
   acaaaacaac cccccctccc gcaaagacct gccccatctc ctttcatccc actgtgaacc      4980
   attgaaatac atatatatca                                                  5000
<210> 5
   <211> 27
   <212> DNA
   <213> artificial sequence
<220>
   <223> forward primer
<400> 5
   gttttcgtcg tttttcgttc ggagatc 27
<210> 6
   <211> 25
   <212> DNA
   <213> artificial sequence
<220>
   <223> reverse primer
<400> 6
   gctctcgctc tcgctattac tcgct
   25
<210> 7
   <211> 23
   <212> DNA
   <213> artificial sequence
<220>
   <223> forward primer
<400> 7
   tgtgtatgcg cgttttttcg ttc 23
<210> 8
   <211> 23
   <212> DNA
   <213> artificial sequence
<220>
   <223> reverse primer
<400> 8
   acctatatat ccgccgctcc gtc 23
<210> 9
   <211> 24
   <212> DNA
   <213> artificial sequence
<220>
   <223> forward primer
<400> 9
   cggcgttggg tatgtttagt agtc 24
<210> 10
   <211> 29
   <212> DNA
   <213> artificial sequence
<220>
   <223> reverse primer
<400> 10
   aattacgaat aaaaaaaaca aaaacgctc 29
<210> 11
   <211> 28
   <212> DNA
   <213> artificial sequence
<220>
   <223> forward primer
<400> 11
   tgacggtttt ttgtttattc ggttattc 28
<210> 12
   <211> 21
   <212> DNA
   <213> artificial sequence
<220>
   <223> reverse primer
<400> 12
   cgaacgcaaa cgtacctacg c 21
<210> 13
   <211> 26
   <212> DNA
   <213> artificial sequence
<220>
   <223> forward primer
<400> 13
   ggttaagttt ttttttyggy gtagtt 26
<210> 14
   <211> 26
   <212> DNA
   <213> artificial sequence
<220>
   <223> reverse primer
<400> 14
   tactccctct acctcccrac tctctc 26
<210> 15
   <211> 28
   <212> DNA
   <213> artificial sequence
<220>
   <223> forward primer
<400> 15
   ttgtggygta gaggattatt agtttggt 28
<210> 16
   <211> 22
   <212> DNA
   <213> artificial sequence
<220>
   <223> reverse primer
<400> 16
   ctaaaaacrc aacccatccc tc 22
<210> 17
   <211> 27
   <212> DNA
   <213> artificial sequence
<220>
   <223> forward primer
<400> 17
   ggaattttat tttgaaattt ttttgtt 27
<210> 18
   <211> 33
   <212> DNA
   <213> artificial sequence
<220>
   <223> reverse primer
<400> 18
   ccccacttca aataaaatac tattaaaaaa aac 33
<210> 19
   <211> 30
   <212> DNA
   <213> artificial sequence
<220>
   <223> forward primer
<400> 19
   tagtgayggt tttttgttta ttyggttatt 30
<210> 20
   <211> 26
   <212> DNA
   <213> artificial sequence
<220>
   <223> reverse primer
<400> 20
   taaacrtaaa aacaacaacc cracct 26
<210> 21
   <211> 5000
   <212> DNA
   <213> unknown
<220>
   <223> bisulfite sequencing of DBC1
<400> 21
   ggtttttatt tgaagttgtt tgattaygtt gttttagatt gattttagaa ttgtattatt      60
   agtagggtag ttgttgttta ttaatagtta tggaagtaga ttttagttgg gggatgatgg     120
   ggaaataaag agaaagatag ggaygaataa aggaaggaat ggggtataga tagaaagaga     180
   gagagagaaa agaaagaaag gaggagaggt aagagatagt gatttggggt tttatatygy     240
   ggtgggtggg agaaggagtg tgtgtggtgg gygtattttt tygtggttag aattaaagtt     300
   agaattgatt tggggaattt gtgtttaaga tttgaatttg gattagaagt taagggaaag     360
   agaaagttgt ttaagaaaaa agggagaaat ttttattagg ygaaaataga aatygttgat     420
   tgggtttgtg gttggagagt tgttygtggt gttgattttt ttttattggg attttagtga     480
   aggttgagaa gttttgtatt ggttgttttt tttttttttt tttttttttt ttagttttga     540
   ttttttgaga tttaggatta ttttggttag ttygatgttt tttttttttt tttttttttt     600
   ttttttgtyg attaaataat ttttttygag tttttagttt attatttaaa tatttttttt     660
   ttattttgtt ttaagatttt ttttttttta attagtttat tggtttttag attgttttag     720
   gtttttttgg tggttatatt ttttttttat tattttttga attaaaaaat aaataaaaaa     780
   tatataatgg taattataaa agtagataat atgtattgag tatttattag gtgttaggga     840
   gtagtttaag tgtttttgta tatattaatt tatgagtagt ttaagtgttt ttgtatatat     900
   taatttatta taattttata ttaattttat tagatgggta gatatattat tattttatta     960
   aatagataag gaattgaggt ttaaggggta agtgatttta ttaagattag atagttatag    1020
   gattttaatt tttttggagt ttgtattttt attgaatttg gtttttttat atattttaat    1080
   attttttttt atgatttttt gtttttttat ttggtagttt taatgttttt tatttttgtt    1140
   taatttttag gggattaggg atttgtattg ttgatttggt ttaaattaga agggaagtaa    1200
   agtaaataaa tatattaaat gtgygttgtg tgggaattat tataygaggg ttttattttt 1260
   tgttttagga agaggtttta tgttagtagt tttagtttgt atttaggttg attgtagagt 1320
   attttgtttt ttatttttat gttttagttt ttgtatttty gttttttttt ygtttttggt 1380
   ggtttttaga gaatttygtg tttttttagt tttttttttt tatattttgt ttaygtagag 1440
   aagtttttgt tttattttgg gaggttaygt gggttttygt ttatatatyg agagaaataa 1500
   atagtgttaa atatttatag agagaygygt agatataaay ggatttatay gggtaatttt 1560
   ygagataaaa tttatattyg atggatttay gyggtygtgg aaatatttgt ygttttagaa 1620
   atatttaggt attygygata tatatagtat agttaygttt aagggtatta ggatttyggg 1680
   tttgygygta tgygyggttt ttttggatgt tygtgygtat agatataata ttttataygt 1740
   tttagattta ygaaattttt tayggtttag ttttagttta ttygggtygt ttttttttyg 1800
   aggyggtttt tygttttttt ttttttygtt tttttttttt tttygtttaa agatgtataa 1860
   aatattttty ggaagtaatt tyggygttta gttttttttt tttygttttt yggtygtygt 1920
   ttttttattt attttyggty gtygtyggtt aagttttttt ttyggygtag ttyggtttty 1980
   gtygttttty gttyggagat ygyggygtat ttggattttt ttttttatty gttagtygtt 2040
   tygttttygg attttayggt tgtaaattga tttggygygy ggggaggagg agagygtagg 2100
   ygagygaatt ygygagagag ggagagagyg agygagtaat agygagagyg agagygagag 2160
   agtygggagg tagagggagt agtgatygtt tttyggagty gggatttatg tttgttttyg 2220
   ggattagyga aggggatttt ayggttgagt atgagttagg ttgttaggag ttaggtattt 2280
   ttaygtttgt agttttygyg tygtgttygg aatgygagtt gtaygtaggg tttttttaag 2340
   tttttatyga gtygaataaa aagygttttt tygtagtttt tygttaaaga yggatattga 2400
   ttttaggtaa ggyggygtyg ggtgtagygt ttygtagttt ygttgttttt ggattyggtt 2460
   tygggtygta ttyggggygt tttygygttt ttttgttttt ttttttatyg gtatttttgt 2520
   tygtttttta tttggtygtt tygtygtttt taagtttttt ttagttttag ggagggggtt 2580
   tttgtgtttg gggtttaaag ggttaattgy gggtttgagt gagtggygtg tgtgttttyg 2640
   ygygttttyg aygtgtgtat tatggtggga atttgatgtg gtgttagtgt gtttgygtgt 2700
   gyggygtygt ttgygtttga tgygtgtgtt ygtggtgtgt gtgtgtgtty gtgtgtgtaa 2760
   gggaggggtg aagagagaga ggttttataa tttatttayg gygygatgtg tgtgtgtatg 2820
   tttgtaygta tgtgtttgta tgtgtgtttt ygtgtgtttt tttaattagg tttttttagt 2880
   ttatayggaa tgggattttt attataggat taygtagtta tygggaaatt ygttgtggat 2940
   ttttttttgg ggttttgggt ttggggtttg gggaggatta tggggttgta gatggtattt 3000
   tatttagttt aatgttggta ygtttgaagg aaaatttttt taaayggtgg aattttgtta 3060
   tattgggatt tatagtttaa tatgaaagag atatggttaa ttttygaggt aaatgagayg 3120
   ttgttatttt aaattttata ttgggtagat tttaagattt tgatgggaat ttggagatat 3180
   tggatagtgg gtgatagaga aagggggaag ttagyggtgg gttttttatt tggggtttgg 3240
   aaagtttggg atagggattt attttgtatt ttygtttgta attaatagag ttttttyggt 3300
   ttttgttggt tttggggagg atttggtaag tttgtatgga ttttttttag gggaaaggag 3360
   aaagygtttt yggggatttg tttatttatt atagttgtaa agggttttag aggaaatttt 3420
   atttggggyg gttgtggatg atatggaagg agatggatgg ggtatttttt taagatagat 3480
   tygttttttt ttttttattt taggygggga agtttttaga gagttttttt ttaaatatgt 3540
   ttttttatgg tttttttgga gttaggggga tattgagaga aggtagagag gtggagaggg 3600
   agagagagag agtaagagyg agagagaggg agatagagag agagagtgtt ttagtattga 3660
   gggagattta taatttgaaa aggggttgtg agtgtggaat ttatgataga atgtggtagt 3720
   aattgattta aattgttgty ggtttgtatt tygygttttt tatttggttt taaaatattg 3780
   ttaagggtag gggggyggtg gaggaatttt agttaggaag gtagtttggg ttaaggtttt 3840
   tttttttttt ttttttattt tttgggaggt tttttaggta ggttttttgg ttattttggt 3900
   ttggtattgt tgggtttggg ttttggggtt agttattgat ttgattttta gttgtttttt 3960
   aaaggtttgt tttatttagt aaaaatgttg agttttattg ttgttagtat ggtttttaga 4020
   ttyggtagtt gttatttttt taaggtaagy ggtagtygtt ttgtttttag gtagggaggt 4080
   taggagaaga taagggttgt gaygttggga tatagttttt tgtttagttt agtayggagg 4140
   ggtygttgaa aagttttttt aggggaaaaa agtagaaata tatttggttt aaaaaatgta 4200
   tatatatatt ttaggaagat atatatatat atatatatat taattatata tatatatata 4260
   tatatatatg tgtttatatt ttggggagag ataaaagtaa atatgtattt gtaggaattt 4320
   gttttagtgg gtgggtttta tttgtaggta tttgtagata tattattttt tgtttagagg 4380
   ttgatatttt aggttgaagt ttttatatag ttatagagaa ttttttttag gaaatttatg 4440
   ttagggtaga gttttttgga ttattttggg tagggggtat tagggttaag gaggagattt 4500
   atttttggga attgttttat tttttttttt ttttaattta tagaygatyg aaattggaag 4560
   gaatattaaa gagtagaaag gtaggatttt gtggtttaaa gagggaatga gaatttttta 4620
   atatttagta gtgggttagt ggtatgttta ggattagaat ttaggtttat taattagtta 4680
   gattaatatt ttttttattt tttttagtaa tatatatttt aggattgtat attgttttta 4740
   tttttatttt gttaatttgt tattatgatt tttattaaga taatygtttg ttttttttta 4800
   ttaaatttta tatttttaga gatgtatttt agggtttttt tatttggaaa tatggttttg 4860
   aagagaaaag atttaatgtt ttaattatta ttttygaaag atatatatat atatatatat 4920
   atatatatat atatatatat atatatatat atatataygg tggtgggggg aggattgaga 4980
   gagagataga gagagaatat 5000
<210> 22
   <211> 5000
   <212> DNA
   <213> unknown
<220>
   <223> bisulfite sequencing of PDE8B
<400> 22
   agaataattt tgaagtatta ttttattttt agaatttttt ttgtagggat gtttgaggtt 60
   tggttaattt ttttttttgt ttaagtttgt ttttttttat tttttttttt tatttttttt 120
   tataaatgtt gattataaga gtatttttta aaaagttttt ttttgtttgt taaatattat 180
   tttagaattt gtttttagga ggatttaatt tgtgtagaaa ttttatgagt aatttgtttg 240
   tttattaaaa aatatagtat agattttttt taggttagtt ggagatggag tttttttgta 300
   ttaaaaaaag agagttaaat tgagaagaga agtaaaggta gaagaataag gttagatttt 360
   ttttgtataa ttgaatttgg gattgggaat gatattttgt ttttttatga aatttagaaa 420
   ttgttttttg aagtttttat ttagttatat ttaagagtgg tttaggaaga aaaaggaaaa 480
   taagaatgaa aaaaaaataa aaaataaatt ttaaaattgg atattttttt gtgttttgta 540
   ttaagttttt atgttattgt ttttgttygt ataagtaatt ttagataaaa ttttttttta 600
   gattatttaa tttatttttt ygtgttattg tgaaaatygg gtaggaatta ttgygggaat 660
   tagtgattga atgggtagtg gtgtttygta gttgtaattt agagtttata agggaaggat 720
   gaatagtagt tattttttta aaggtatttt tttttttttt tagtatagtt tagtttgtgt 780
   atgttttatt aaggagaatg agggaaggtt ttgttgattg tttagtttag agtgtgagga 840
   atagtagttg atatgggygg ttggayggga tagaaattag tatgaatatt tagtgatatt 900
   taaggaagag gtgaaaatgg gggagaaagt taagaattat tagatagttt ttttttgttt 960
   ttgttttgga ttttagggga aygtatgtgt ttaagggtgt tttttttygt agggtttttt 1020
   attttttggt ataaattttt aaaygatgag tagatatgga atgggttttt tttgaagatt 1080
   gggttattat ttaatttagt tttyggagta ggyggtgggg ttgggggaag aggayggaat 1140
   tatgaggttt ttttattatt tttygggtgt gttgagaggt aagtaaatgy ggtgggtggg 1200
   tttgggttgt agggtttttg ttatttyggg aggggttttt tggtgtttag ttttgggatg 1260
   tgtgaaaatg tgttggtgaa aagygagagg tttatatagt tttttagggg aagaggggtt 1320
   ggggygttgg gggyggygty gggatgagtg tagaagagay gaggtttttg gatagyggag 1380
   gaggagggga gggygtygag gygyggtgtt agttgtygyg tataggggtt tygyggygga 1440
   gtygagtygy gggtaygttt tgttttgtyg ggagagttty gggagyggyg ggaggggygg 1500
   aggggygtag tggggttygg gyggttgygg tygyggagty ggggtatttg aggaggaagg 1560
   agggtgggag ygagggaggg aggggayggg ygtagatyga aagtggggaa agaaggtgta 1620
   ggtaggyggg taggygggyg ggygttttgg tttagggtyg ygggtgyggg agttyggyga 1680
   ggtygagttg ggyggyggyg ggggtygygt ygagggagga ggggaaggyg gaggygyggg 1740
   gagygtgttt ggggygtygy ggygggaggg gtggyggtyg ttggtgygyg yggggygttg 1800
   tgtatgygyg ttttttygtt yggggaggaa gatggtttaa aagggaaagt tggggtgayg 1860
   ygygyggttt tyggaggtty ggyggggggt atygyggtta gttygaygga gyggyggata 1920
   tataggtygg ggggygygta gttygggygt ygtygyggty gtttttttat tgtaggtggt 1980
   agygggtgyg ttgggtttyg gyggtygygg gygygggygg gygygygggg gagttyggty 2040
   gagggatggg ttgygttttt agtatttatg tttygtagag yggygtgatt tattgtyggg 2100
   attyggayga gtttagttyg tttygttaga ttattagygt gtygtagggt tyggyggtat 2160
   ttttgttygg ttttttygtt tagatygayg tygtygaygt tattttttyg agtygygygt 2220
   ygggattttt tagygtagtt ygygttygta gggttygtat ygagttgggt agyggtagta 2280
   gygygggttt ygtagtttty gtygygatta ttagtagggg tyggaggygt tattgttgta 2340
   gtagygtyga ggtygagatt tagatttgtt atattagygt gaaggtaaat gtttygygtt 2400
   ggtataygty gtgggggtyg ttygtttygt yggyggggtt ygtaygggta gggggtttyg 2460
   gyggagttgg gtgatygtga ggyggttggt ttggagaggt tgttattaag gaggagttta 2520
   ttttttattt gtggagatga tgggagttta ggaaatgtgg ttagaaaaag gtttttggag 2580
   gggttttgga agygttttta gttggttttg ggggattggg yggggaaggg agygtagaag 2640
   gaagtaggtg ggttggtttg tttttttttg agggtaggaa ggttgtggtt tggtttatgt 2700
   aggaagaggg gtggggatta ttgagagtat tyggtggtta gttttgttga atgaaatttg 2760
   agtattgagt tggatttgyg tgttttgtag gtgattggtg tagttgtagt attaggatag 2820
   atagtgtttt atatttygat ttttatttgg gattattagt taggttggag ttttagtata 2880
   ggaatygagy gtagggattt gtgaatgaat gagtgttygt gttttaagag atgtgggaay 2940
   ggagtagagt ggaatttgtt gtttgttatt gtaaygtttt tttgggttgg ttgtatttta 3000
   gatagaattg agagaatygg gttatgagtt yggagtgtta gtagagttat ygtgagggga 3060
   ygtggttttt agtgtagtat agttgtttga ggatgatttt gtatatataa ttgatttttt 3120
   tagagagtgg gattttgagg aagtggaaaa aattgtttag atggttaaag tagtygttaa 3180
   atatttattt tttaaagata gaagaaaaat aattatttaa atagtgtttt ttygtatgtt 3240
   tttaaagtat ygttaaattt aagaggtttt ttattgtgta aatttgggta ttgggttttt 3300
   ttttttttta gtaaataaga taataatggt atattttatt gtatagagag aaaaaaatat 3360
   ttttaatgtt agatagaatt atagttttta tttggttaat aggttattaa gaattatttt      3420
   atggattatt tgttaaagat atatttgtat ttttaatagt taaaaatttg gtttttattg      3480
   gatgaaggta gtttatagtg gaggagtgag gagaaggata atatgttttg gttttaattt      3540
   ggaagtttaa aagtttttta atgaatttat ttttttttta atagtatttg attgtttaat      3600
   atgaagtttg attgatgttt gtttgtggtt gtagtgtttt tttggtagta attataatta      3660
   ttattatagt aaaaatatyg tttattgagt atttattatg tgttaggagt aagttttttt      3720
   ttttaattat tgaatgttaa tagtgtgatt ttagtatagt atgtttgaag attagagttt      3780
   aggatttatg atagtataaa tttattagtt gtttattygt tatagaggta tgtgttgaay      3840
   gttatatttt gtttttgtta agtttttttt tttttttttt ttttgtttat ataaagttag      3900
   ttttaggagt aatttttagg gatgaaaatg ttaaatttgg tgaaaatatt aagttgggat      3960
   attattagta aagattattt ttttgtttga yggttgaaga tttgaagtag tagtttaaaa      4020
   ttgtgatagt ttatgaattt tgtgatattt tttttggttg ttttagagaa attgtgaatt      4080
   tttgtttttt agaatattta taattttagt ttagtatttg gtaattttag ttattttatg      4140
   tttgtttagt agaagaaaga atattgaatg aatttatttt gtatattttt aatatttttt      4200
   ttaagaggtt gtttagagat taaatgaagt aaaaattgaa gggataatga gttttaatta      4260
   ttagtagttt gtataaattt gtattaattt tattgttatt tggtttaagt attaaaaggt      4320
   ttgatttggg taggagttta atttattttg ttttattaag aagattttta aataagagtt      4380
   taatttattt taatttatta ttgggaatgt tggaaatgaa atttttggtt tattgattta      4440
   atattatttt gtattgtgtg gattttagaa agtatatatt tgaagtgaaa tttgagatta      4500
   gagttattta tgtaaatttt atagaattat tattatagaa ttgttttaat atatttaata      4560
   gtattttttt aggtttgtga ttyggtgttg tattgttttt ttttaatygt aaagtttata      4620
   tgttttgtgt ttttttgttg ttgagataat tttataaaaa ttttatattt atttttttgt      4680
   agattaagta tttttttttt gtygtaagag tttgagatag ttgttaggta ygtggtggtg      4740
   ttttattttt ttttaaagta tataggtgga aaaataaaat ttttattatt tggttttgtt      4800
   tatagtggtg gaaggttgta atagttttta tttttttttt ttggttattt tatattaatt      4860
   atgaagtgta tttttattat ttatttggtt tttggtattt tttagagtgt aaaaatgtgt      4920
   gtttgatttt tttttttttt ttttttagta ataatttatt ttattaagtt tttagaatga      4980
   tgaggtatta tgatgttaaa                                                   5000
<210> 23
   <211> 5000
   <212> DNA
   <213> unknown
<220>
   <223> bisulfite sequencing of PTPRR
<400> 23
   aagaaaaata gatttattgt aagtgggata taattatttt tttttaattt ttagttttgt 60
   agtattttta gtaatagatg ttagatgaga agagttattt ttttaagttt tatagaatgt 120
   taaataaatt attttaaagg ttgatttagt aagaatttta aagagttaat tgttagtatt 180
   gaattttgtt aattattatt tttatttatt tatttttgag tatttatttt gtatgaggta 240
   ttgttttagg gagttgggat atattaataa agttattaaa gttttttaat attggagttt 300
   aaattttgtt ttgygggggg gagtagggaa atagatatta aataatagat tttataagta 360
   attattttgg tttgttagaa agtgataygg gaaaagtaaa ttggaggata gtgaattagg 420
   aataggagaa gaggttgtag tttagagtgg tttgggatga aatgatagtg ttatagaaat 480
   ggagtgtgtt ataygtatgg gggagtaggg ggtggagtay gttgttagag aggggttaga 540
   tatggagata tgtttttatt tttagtagag aaggtttgtt aagatattaa aagtatattt 600
   tattgttttt tttttttatt tgttggagtg ttttttggtt ttgtttttta gagttgaaga 660
   tgatataaat tataaaagaa ataaatggta tygtttgggt tttttgattt tataaggttt 720
   tatttaattt aagttttggt ggatagttta ttttttatta gttgttttaa tttatagagg 780
   atggagaaag aaagtattga atgagatgtg tagtttaatt taatgggtat ttgttagttt 840
   taattatttg gttaggataa ttttatttaa gttaatggaa tataaaattt agtagttttt 900
   atggagattt taatgtgttg tagaataaat tatagataaa tgaattgttt gaaagaataa 960
   tatgaggtay ggttaaataa gtgttgtgga agagaaagat tattgtaatt tagagttagt 1020
   tagagttagg gttttttgga aaagtgagat taatattata ttttygaaaa ttagtgtggt 1080
   ggtggtgtaa gtggaggaaa atgggaaaga ggtaatagtg tttataaaga tatagaggta 1140
   gaaagggtag aggttttggg atagttaaaa gagaagttta gttgagggat tttttgattt 1200
   gattagtagt aattagttat gattaataag gttttttata ttttaaagat ttagatggag 1260
   ggataaaaaa ttatttaatg gtagattgtg gtagtttttt tagagatata gagttgggty 1320
   ggatgagttt aggtattgay gtgatttatt attttttatt ttaaagagta aaagggaaat 1380
   taaagttaat tatttttayg aaataaaaag gtgttttttt gtgttttaat tatatggata 1440
   tattttatta gtttaaaagt atttttttat ttttttttgt tattgtgagg atttgagtta 1500
   gaagaaagtt taaatatagt tattgagttg gaaagagtgg aaagagaagt aaagaggggg 1560
   aagttgtagg aaggaygaag ttatttttaa gatatatggt tattgtttat attaagtaag 1620
   ttgttttggg aaygtttttt tygagtagtt agaatgttta gtagtggaag atatttttat 1680
   ttttgtaggy gagttttggg aagttggtta atttgtaaat gttaattttt agtagtgagt 1740
   tyggtttayg tgtaaattaa gatttgggga aagagtaggg tgggtggtat ggttgataat 1800
   gttattagtt tttttttttg atttttgtgg tygtgttttt atttattttt atttagttat 1860
   attttatttt yggatttgtg atggaygttg ggtttttagt aattatagta agtgtttttt 1920
   tygtattttt ttttttttta tttttatttt tatttttaat tattatttta gygatggagt 1980
   ttattttgtt ttaagtygty gttaagatty ggagaagygg aattttattt tgaaattttt 2040
   ttgtttygtg agggtyggyg ttgggtatgt ttagtagtyg yggygttgtt gttgggttgt 2100
   tgggttggyg yggagtttat tttgtygttt tygttttggt ttttgggygt ttagaaggtt 2160
   aggtatttgt ygtttttgag ygtttttgtt ttttttatty gtaatttttt attgtttttt 2220
   tttttttttt tttttaggga ggttgaagtt ggtgttggtt tttgtyggyg ttatagattg 2280
   attgttttgt aaattttagt ygaggatttg aatttyggag attagaagat ttttggyggt 2340
   ggtttttttt aatagtattt tatttgaagt ggggtygtgg tggagttttt tttttatttt 2400
   ttaatgtaaa tattatgygg agagtagttt gtttttttgy gttgtgtttg ttttttaatt 2460
   tttaygttgt aggtaagggg ttgttaggtt tagagtygga gttgtgtatg agatgggaaa 2520
   ttgtatatgt ttaaggattt taggaaaagt ttgttttgyg aagaaagttt ttttaaaaag 2580
   gtaaaatagg atagtatttg atagggaagg ggtgagggag tygtgtattt gttggaaaat 2640
   tgaggtygaa aatttaattt aaaattagtt tttgattttt ttttatttta taygaaaata 2700
   gtgaatattt ttaatatgaa tataggtttg tttttttttt ygtttttttt tygtttagat 2760
   tttttgtttt tgtttttagg ttatgtattt aattgttaat tttttaggag agggataaga 2820
   tattttgtta gatgtaattt tttttattgt agyggttatt atatttataa ggttttttgg 2880
   tttagygagy gtttatagga aggtattggt tgtaatttga tggattatat tttygtttaa 2940
   aagatygaaa tatatggttt atattagtga tgttgtatta ggtttttttg gtttttgttt 3000
   ttgttattyg aattttttaa gttaataaga ggaagaaaag gttttaaata gaattttttt 3060
   gtttttttta gtatygtggt tagygttygt tggtatatta aaaaaaaaaa aaaaaaaaaa 3120
   aaaaaaaaaa ataattatag ttttaaagtt gataattttg ggttaggtat tgtttttygg 3180
   atagatttga taaggtgtaa ttaagagtaa tagtgagatt gtttttaaat ataaatagat 3240
   ttgaggtatt agaattaata ttaagtttyg tgaggaagta aattttggga agtatttgag 3300
   agatattata tgtggtgttt ttgttttttg tatatatagt atttttattt aatgtagata 3360
   gatttgttag gttgaatttt ttattattgt tatatatata tgaattaaaa taaaatatgg 3420
   tagggagatt ttagaaattt tattgtgata gtagttgttt atgtagtagt tttgtaataa 3480
   ttagagayga ggtatttttt ttgagtgtgt tttttattta attatatata tttatttatt 3540
   tttttaagta atatttgttg agtgtatatt atgttttagg ggtaatgagt aaaaataatt 3600
   gttttgaaat tataattttt gtagtatttt ttttaaaaga gtgggtgaat tttatttttt 3660
   gtgtataata gtaggagtga aatatggtat ttttttattt tagttaaygt tattatttaa 3720
   atgtattatg aattttttga gtaaataatg tataggatta ttaaagttat tattaaatat 3780
   atatgttaat tgttataatt atgatgttat attgtggagt ttttaatagt tttttggtat 3840
   attttgagaa gtagaaaata gttatgattt gattattaaa ttatttgtaa atagttgtag 3900
   tgaattattt ygttattata tataattttt taatgttgaa atgtgggatg aaaatatata 3960
   tattgggttg agttttattt atagtattgt ttaataatag attgatataa atatttatta 4020
   tagaaaaatt ttaattttta ttaaggtagt aatgatttag ttggtttgtt aataatatta 4080
   gtgtagttat tttttaaaaa ttaatatata aaaaagttta aagattatat ttgatatatt 4140
   ttgagtaatt atattttaaa agttataaat aaattgaagt atgtgaaaaa tatgttttga 4200
   tttattgtag atgaatatty gggatgagaa aaatgaggat ttttgtttat tagatgagaa 4260
   atttagaagt ttgtagtttt agattttatt tggagttttg ttaaagggta tattattaaa 4320
   gttaatttaa tttattttta ttttaatttt ttaatttgta aaataaagat aaaaagtaag 4380
   attaattaat taattttaat ttaataagaa aatgtattta aggatatttt tatattttaa 4440
   ttgtaagata ttatttattg tatttatatt taatatagta aaatatattg gtgttttatt 4500
   atagtagatt gtaagtttaa tattatagtt tttttgttaa ttttatttta aatgtatttt 4560
   aattttaaat agtaatttgt gggttataag ttgttattag attaaaattt attatagtta 4620
   tttaataata ttgttagggt tttttattag aattaaaata aatatagttt tttggtttaa 4680
   gtttttatat atttggagat taagaaaata agtggatggg atgataaaga ttaagaaatt 4740
   aagatattaa tttttatttg ttgaaggtgt gagtttgtta tttttatgga agattttttt 4800
   tttagaatag aggttgattt tataaagaag ggaaatgaat tttatatatt taggtttttt 4860
   taattgtaaa gttaattgat ttattttggt taygtttagg gtattagtaa ttgttttatg 4920
   ttggtggaat tgatgatatt ttataatgtt ttagagatgg gaggaatgtg ttaaygggat 4980
   gtaagtgttt tttttagtta 5000
<210> 24
   <211> 5000
   <212> DNA
   <213> unknown
<220>
   <223> bisulfite sequencing of ZNF582
<400> 24
   gaygattttg aatgattttt gttttttagt atttatattt ttgtgtagtt tttatttatt     60
   tagatttata gttgattttg tgattaagag tataatggtg tatgatttta gaggtttggt    120
   ttagagttag aagtggttat tttattattt tttaaatttt tttttggtag attaatgaat    180
   gtgagaaagt aaaaatggta tagaagaaag ttagtttgag agatggttgg ttttgtattt    240
   gtttggtttt tttttttttt tttttttata agtaaatatt gaygtatgta tatatagata    300
   tatttaatat agtgggatgt taaagggaaa ttagagttat tygttttttt ttaagattag    360
   agaatatagg aaagatgatg attttttgta tttatttttt ttttttgaat tygtaatatt    420
   gttttgaatt ataaataagg ttaagtagga taaatttata attgtttttt taattgttyg    480
   tttataagtg tgaaatttaa ttatttttgt tgaagttgtt ttttaatatt agtaattagt    540
   aataattaat ttttgagttt tgtttatatt attaaattat tttatttgat ttttaggata    600
   aagttgtaag tttagtatta tatttttttt tttatggata aaaaaaaaaa aataaaataa    660
   aatygagata ggtaygaaga aattaataaa tggttgaaat gttatttgaa tttatatttg    720
   tttaatttta aaagtgtgtt ttttattata ttatygtatt tataaggaaa atgtagtttt    780
   tatttttttt tgaagaatgt gtaaagaggt aatatggtgt gttttggaaa aagtattttt    840
   gtagaaagaa agtattagtt ttagttatat tagtattttt atttagtagt agtagtatta    900
   ttttatatat tagttatttt tttttaataa tttaatgaat tagatgttat aattattttt    960
   atttttaaat gtggagatyg aggtatattt ttaagattgg agtttaggta ttttggtttt   1020
   agttttagag atggttaagg gtttatattt ttaattattt attatattat agagtttatt   1080
   aggtttgagg gaaataggat taaattaaaa gagttattta ggattttagt ttttatttaa   1140
   ggataaattg ttttatttyg gatagggaga gttttygtat tttgagattt agtataatag   1200
   gttttgatyg gtatttggta ttyggatttt taattatatt ggattatatt ggttygggat   1260
   gtgtaaagtt tagggttttt tatatttgat gatattaaag tygtttaaaa ataagagaga   1320
   attaataatt atttayggyg gtttgatatt tgtttaagag atgtygtttt ataaaatttt   1380
   tttatatttt tataaygttt ttattttgyg ttttttttta taatttatat ttaatttatt   1440
   atagatgtaa tgtttaaaat tagttattag ataaattttt aygtttttaa attttaaggt   1500
   tttttygaaa ttttttggta aaattgttgt tttayggaaa tgggaaygta ayggatgagg      1560
   taatttttta tagtygtata tagttgtgta tttatygtta aayggtttta gttatatatt      1620
   taaygattta ygyggagtta gaagttatta ttatatattg ttaaaattay gtatatatag   1680
   tgayggtttt ttgtttatty ggttattygt ttataatttt tygttagaga attataygta   1740
   gatagtatat ttagtattat agattttagg aagtaattta gggattygaa tataygaata   1800
   gtatttttty gygtattgyg taggtaygtt tgygttyggt ttattttgaa atatygygag   1860
   attyggtttt aaggtygggt tgttgttttt aygtttaaag attatgtttt tyggaagata   1920
   ttgyggygty ggttttatta tggygtagta tyggtgtgtt ttgtgygttt gygttatttt   1980
   tyggttgygt ayggygaatt tatyggtaty gtggtggaag ygygttttgg gttgtygggg   2040
   gygyggtygy ggtggtattt ggattygagg aggyggtagg tgagaggttt yggagttttt   2100
   taggygtttt ggggtttagt aggagttggt gttygggtyg gttgggtttt aggtttgaga   2160
   agaaygtaga ygtttygttt tatygtygtt ttgtggtttt atyggygtga gattatattt   2220
   ttygtyggtt ttygyggygt gygttttttg gygttttttt tttgttttta gttttatagt   2280
   ttaggtgtat ggatttttta ggtgggtgyg taggggtttt ygattttttg aaattgygga   2340
   tgttttgtat ttattgttat gygtgttttt tttttttttt aatttgagag gaaattttgt   2400
   tttatgaggt ttttagagag ttataagatt ttagaagatt tagaatygtt agtttagaaa   2460
   aatttatatt tgggaatatt ttatatgttt aaattttatg tttagtaatg gggaaatatt   2520
   tgagtggatt ataggttatt ttttgtaaat aggatatygt gtagttaata taaataatgt   2580
   ttataaaagt ggttataata aggaaaagtt gtttattata ttatattaag taatatttat   2640
   aattatttta tttataygtt atttagaatt aaatgtttgt aatgtagtag gtattttata   2700
   tattttttta tagagtattt tttgtttaat agtgagttta agaattggtt aaatgataat   2760
   attgygaata tttgggtgtt ttttttttta atttttttgt tttgttttgt ttttagtttg   2820
   tgtgttagtg agtttgattt ttttttgttg aatgttttta aaggatttag tagttaatat   2880
   ttttgtttgt tagtttttga tagttatttt tgatagttat ttggtatttt attatatggt   2940
   tgtgttaaaa atgatttaat tttttggtta tttgtggatt tgtttattta ygtattaatt   3000
   tttagttttt ttatttagta aatattattg gatatgtagt tttgtgtatt gttgtgtttt   3060
   tatggaagga tatatttatg tgttgataat ggagtattgg gygtgttgtg tgtaatttgt   3120
   tttttattgt tttttttaat tttattttat ttgttgatgt aaggtatgtt ttatgaagtg   3180
   gtatgtataa agttttagtt tgggttgtag agttttttgt atatttggyg gttgggaaaa   3240
   gagttaggta gatttttaaa ggtgttatat ttgttgtagg agagagttaa atggtttagt   3300
   tttggaaagg ttttatttta ggttggggtt atgtaaatga tgtaggtttt ttatgttttt   3360
   tggtttgagg gaagtttttt ttgtttttga ggtaatatgt gggttttagt tattagaata   3420
   gtttttagta tttttgtttt agatttgtat ttgtagtaaa atagaattat tggatgttta   3480
   ggggaaatgg gagggaaagg attattggtt tttttttgtt ttgtagtagt taaagttggt   3540
   tttttggggt tgggtgagga tttttttgtt tttttttttt ttttattttt ttggtgggta   3600
   gagggtaagt aggtggggat ttgtaatttg gtattttagg aattgtgagg taagtttagt   3660
   tttttatgtt tgtttttttt ttttttagtt ttatygtygt aggattttgt ttttttttaa   3720
   gagaggaatt agaaggagga tttattagtt tttgaaattt taaaagttat gttttttgtg   3780
   agtttttaaa ttttataaat atttgttttt tttattttga aattttttat ttgggtttta      3840
   tttagaaatt tggtttttag atttttttat tttagggaat aaaaggatta ggtaggagtt      3900
   ttttttttta ttgttttttt ttttttgata aatgtttata gatttatttt ttatagtatt      3960
   ttgtattttt tgatggtttt ygtttgttag gaggagaaat ttattttttt gtatgaaatg      4020
   agagtatttt ttgtaagaat tgaagtttga agagatggaa tgaatgttta ggagtaatta      4080
   tatatagaaa agtaggggag gtggtgttgg ttggttttag ttttagygta tggtatagtg      4140
   ttttgttttg ggaatgatta atgattggat atgtaattga ggtttggttg ggaatttatt      4200
   aaaaatttga tgttagaatt aaagtttagt ttgaygttgg atagagtatt tgaatttggg      4260
   atatttttta ttagtttttt tagtttaaga ggaagaaaag tatagagttg aggatggtga      4320
   gygatgtagt aagtgagatg attatttagg tgggtgagtg gttagggttt ggaaaagttt      4380
   tagagatttg atttaattty gggatttaaa ataagaggat gtttgagtta gttgttttat      4440
   ttgatagaaa attgtgtatt taataaaagt tatttttatg gtttttttta tgaagaaaaa      4500
   tgtttgtgtt atgttttaat ataatgatga atttttattt ttaagaaatt tttgatttat      4560
   atgaaaattt tgtatttaag atagaaaata tgtttttata attttaaaaa ataaaatatt      4620
   tggggaaaat ttattagaaa tattttttgt tgttattgtt attttaaaag atagaagtga      4680
   taggttttat tttgagaatt tattgtatgt taagtataga gtgggtagtg tgttttatta      4740
   tattgttttt taataataag gaaaatttta tttagtttga ttttaatttt tattttagat      4800
   ttgtatatag agtattgatt tgtagttaaa aggaatgttt gagaataatt tgattatttt      4860
   gttttattta tttttatttt ttttgttatt ttttatattt agttttattt tttttgttta      4920
   ataaaataat ttttttttty gtaaagattt gttttatttt tttttatttt attgtgaatt      4980
   attgaaatat atatatatta                                                  5000

## Description

### BACKGROUND OF THE INVENTION

### 1. FIELD OF THE INVENTION

The invention relates to the cancer screening method, cancer screening biomarker, and use thereof. In particular, to the cancer screening method using methylated DNA as the biomarker.

### 2. DESCRIPTION OF THE PRIOR ART

Cervical cancer has been one of the main causes of death in females worldwide and in Taiwan. Based on the statistical survey by the World Health Organization (WHO) in 2002, cervical cancer was the second major disease responsible for the death of women worldwide, second to breast cancer. Regular cervical cancer screening is the best way to prevent cervical cancer. Conventional cervical cancer screening includes two approaches: the most commonly used Pap smear, and human papilloma virus testing (HPV testing). Pap smear consists of sampling secreta from cervix uteri, examining under microscope whether there is cancerous pathological change in the exfoliated epithelial cell, so as to detect cervical cancer early. On the other hand, HPV testing consists of examining whether human papilloma virus (HPV) is present in the specimen by using polymerase chain reaction (PCR) or Hybrid Capture.

There are, however, many undesired properties of Pap smear. For one, it requires sampling by a physician, and analysis by a medical examiner/pathologist, which is a high cost of manpower that poses difficulty on promoting the test in many developing countries. Also, Pap smear has a high false negative rate which delays diagnosis and proper treatment prior to cancerous pathological change. As for HPV testing, although it is highly sensitive, it tends to create a high false positive rate, which not only leaves patients worry in vain, but also wastes much medical resources in examinations follow up to those false positive patients. Accordingly, one of the important topics in promoting cervical cancer examination relies on increasing the accuracy and convenience of cervical cancer examination method.

Genomic deletions have long been considered to be an important factor in tumorigenesis. For a long time, we have been accustomed to the idea that the coding potential of the genome lies within the arrangement of the four A, T, G, C bases. The two-hit theory proposed as early as in 1970s indicates concomitant mutations or deletions of some homologous tumor suppressor genes may cause or predispose to cancer development. However, additional information that affects phenotype can be stored in the modified base 5-methylcytosine. 5-Methylcytosine is found in mammals in the context of the palindromic sequence 5'-CpG-3'. Most CpG dinucleotide pairs are methylated in mammalian cells except some areas called "CpG island." CpG islands are GC- and CpG-rich areas of approximately 1 kb, usually located in the vicinity of genes and often found near the promoter of widely expressed genes. Cytosine methylation occurs after DNA synthesis, by enzymatic transfer of a methyl group from the methyl donor *S*-adenosylmethionine to the carbon-5 position of cytosine. The enzymatic reaction is performed by DNA methyltransferases (DNMTs). DNMT1 is the main methyltransferase in mammals, and is responsible for the post-replicative restoration of hemi-methylated sites to full methylation, referred to as maintenance methylation, whereas DNMT3A and DNMT3B are thought to be involved primarily in methylating new sites, a process called *isolated* methylation.

Loss of methylation at CpG dinucleotides, i.e., general hypomethylation, was the first epigenetic abnormalities identified in cancer cells. However, during the past few years, it has become increasing apparent that site-specific hypermethylation, e.g., some tumor suppressor genes, is associated with loss of function which may provide selective advantages during carcinogenesis. Dense methylation of CpG islands at promoter regions can trigger chromatin remodeling through histone modifications with subsequent gene silencing. Therefore, in addition to chromosomal deletions or genetic mutations, epigenetic silencing of tumor suppressor genes by promoter hypermethylation is commonly seen in human cancer.

Epidemiologic studies have recently shown the correlation of serum folate level, a major source of methyl group, with the infection and clearance of HPV. Genetic polymorphisms of enzymes in the metabolism of methyl cycle were also reported to be associated with the development of cervical intraepithelial lesions. As the concept of epigenetics evolves, studies exploring the association between DNA methylation and cervical cancer are also booming. Studies of DNA methylation in cervical cancer are accumulating, which showed the potential of using methylation as markers in cervical screening. With the nature of the interface between genetics and environment, the prevalence of methylation in tumor suppressor genes varies in different genes and different populations. The concept of methylator phenotypes with different disease behaviors was proposed with controversy. The methylator phenotype of cervical cancer and its interaction with HPV genotypes still remains unknown. What genes are specifically methylated in cervical cancer and how many genes are required to achieve clinical application will remain a blossoming issue in the coming future.

San Jose-Eneriz E et al., "Downregulation of DCB1 expression in acute lymphoblastic leukaemia is mediated by aberrant methylation of its promoter", British Journal of Haematology, vol. 134, 2006, pages 137-144 discloses the downregulation of DBC1 in leukemia by aberrant methylation of its promoter region, establishing the methylation rate of DBC1 as diagnostic criteria for leukemia.

Izumi Hiroyuki et al., "Frequent silencing of DBC1 is by genetic or epigenetic mechanisms in non-small cell lung cancers", Human Molecular Genetics, Oxford University Press, Surrey, vol. 14, no. 8, 15 April 2005, pages 997-1007 discloses the silencing of DBC1 in urothelial cancers and lung tumors through hypermethylation of a defined CpG region of DBC1, postulating that the methylation status of DBC1 is suitable for cancer prognosis.

Groenbaek et al., « Frequent hypermethylation of DBC1 in malignant lymphoproliferative neoplasms », MOD PATHOL., vol. 21, no. 5, 2008, pages 632-638 discloses hypermethylation of the promoter region of DBC1 in various lymphomas and lymphoproliferative malignancies, making DBC1 suitable as diagnosstic marker for those cancers.

The inventor of this application had filed relative patent applications in Taiwan (TW Pat. Pub. No. 200831900) China(CN Appl. No. 200810094659.2) Malaysia(UI20085354) and America (US Pat. Pub. No. 20080311570) (Hereinafter referred as prior applications). Comparing with prior applications, the inventor had found some novel cancer screening biomarker and cancer screening method using the same.

### SUMMARY OF THE INVENTION

One object of the invention is to provide a cervical cancer screening method as the first line cervical cancer screening.

Another object of the invention is to provide a cervical cancer screening method, characterized in that said method can be used not only in the first line cervical cancer screening, but also in the second line cervical cancer screening to assist HPV testing or uncertain smear result in order to achieve more accurate cervical cancer screening effect.

Yet another object of the invention is to provide a cancer screening method, characterized in that said method can be used in the screening of cervical cancer as well as in the screening of other cancer (for example: ovarian cancer, colon cancer and the like) to assist the diagnosis of abnormal specimen.

Another object of the invention is to provide the cancer screening biomarker, or a kit containing said biomarker to screen the risk of having cancer. The cancer screening method that can achieve the above-mentioned objects of the invention comprises of detecting the methylation rate of the target gene (biomarker) of the test specimen and use the testing result as a screening index to indicate the risk of having cancer, and said method comprises following steps:
step 1: providing a isolated test specimen;
step 2: detecting the methylation rate of CpG sequence in at least one target gene within the genomic DNA of said test specimen, wherein said target gene comprising DBC1, PDE8B, PTPRR and ZNF582; and
step 3: determining whether there is cancer or pre-cancerous pathological change or not in the specimen based on the methylation rate in the target gene, or using said methylation rate of target gene as prognosis marker;
wherein said test specimen comprising a cervical scraping, a ovarian cancer tissue, ascites, blood, urine, feces, sputum, oral mucous membrane cell, gastric juices, bile, cervical epithelial cell, and post-surgery cancer tissue;
wherein testing methods for the methylation state of CpG sequence in said target gene comprising methylation-specific polymerase chain reaction (MSP), quantitative methylation-specific polymerase chain reaction (QMSP), bisulfite sequencing (BS), microarrays, mass spectrometry, denaturing high-performance liquid chromatography (DHPLC);
wherein the target gene DBC1 has the nucleotide sequence as depicted in SEQ ID No: 1;
wherein the target gene PDE8B has the nucleotide sequence as depicted in SEQ ID No: 2;
wherein the target gene PTPRR has the nucleotide sequence as depicted in SEQ ID No: 3; and
wherein the target gene ZNF582 has the nucleotide sequence as depicted in SEQ ID No: 4.

In addition, the above-described screening index and screening method can be used further in the screening of cervical cancer, and colon cancer.

The invention provides further a ovarian cancer screening method, characterized in that said method tests the methylation state of the target gene of test specimen, and uses said methylation state as a screening index to determine the risk of having ovarian cancer, and said method comprises following steps:
step 1: providing a isolated test specimen;
step 2: detecting the methylation rate of CpG sequence in at least one target gene within the genomic DNA of said test specimen, wherein said target gene comprising DBC1, PTPRR and ZNF582; and,
step 3: determining whether there is ovarian cancer or cancerous pathological change or not in the specimen based on the methylation rate in the target gene, or using said methylation rate of target gene as prognosis marker;
wherein said test specimen comprising ovarian cancer tissue, ascites, blood, urine, and post-surgery cancer tissue;
wherein testing methods for the methylation state of CpG sequence in said target gene comprising methylation-specific polymerase chain reaction (MSP), quantitative methylation-specific polymerase chain reaction (QMSP), bisulfite sequencing (BS), microarrays, mass spectrometry, denaturing high-performance liquid chromatography (DHPLC), and pyrosequencing.
wherein the target gene DBC1 has the nucleotide sequence as depicted in SEQ ID No: 1;
wherein the target gene PTPRR has the nucleotide sequence as depicted in SEQ ID No: 3; and
wherein the target gene ZNF582 has the nucleotide sequence as depicted in SEQ ID No: 4.

Term "test specimen" refers herein to isolated test specimen, and said isolated test specimen comprising the above-described cervical scraping, ascites, blood, urine, feces, sputum, oral mucous membrane cell, gastric juices, bile, cervical epithelial cell, post-surgery cancer tissue, or other suitable specimen. The inventive cancer screening method is useful to test the methylation state of target genes in said isolated test specimens, and uses said methylation state of biomarker as a screening index of various types of cancer. The cancer screening method and the screening index of biomarker provided by the invention can be employed by the researcher to perform the test in the laboratory.

Wherein, "the risk of having cancer" refers herein to the percentage of being cancer, the risk of getting cancer in future, or the presence or not of cancer.

Wherein when the methylation state of target gene (biomarker) being higher compare with suitable control, the risk of having cancer would be higher.

According said screening method, the invention provides further the cancer screening biomarker or a kit containg said biomarker to screen the methylation state of target gene (biomarker) in isolated test specimen for determining the risk of having cancer.

These features and advantages of the present invention will be fully understood and appreciated from the following detailed description of the accompanying Drawings.

### BRIEF DESCRIPTION OF THE DRAWINGS

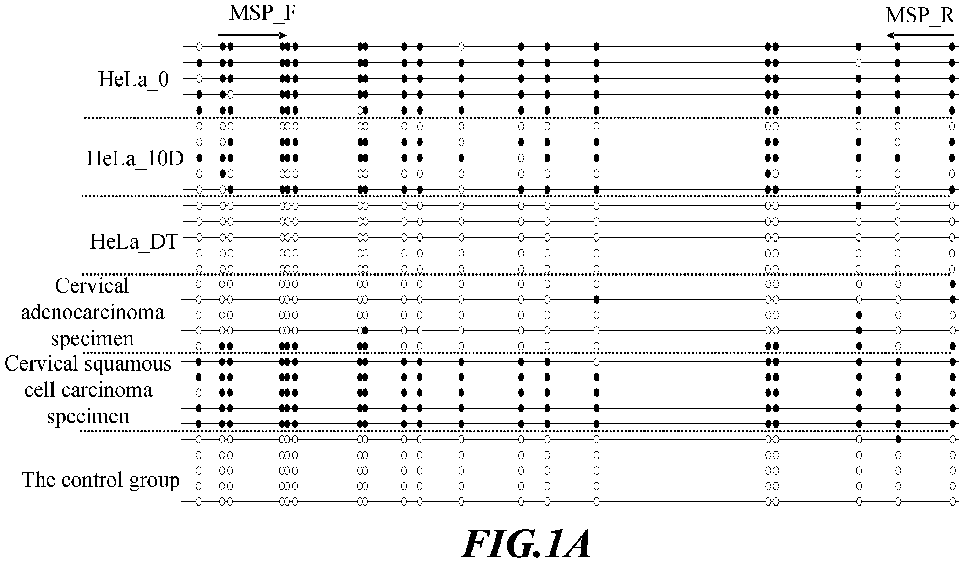

Figure 1A shows the result obtained by performing bisulfite sequencing (BS) analysis on various cervical specimens using a target gene PTPRR in the inventive cancer screening method;

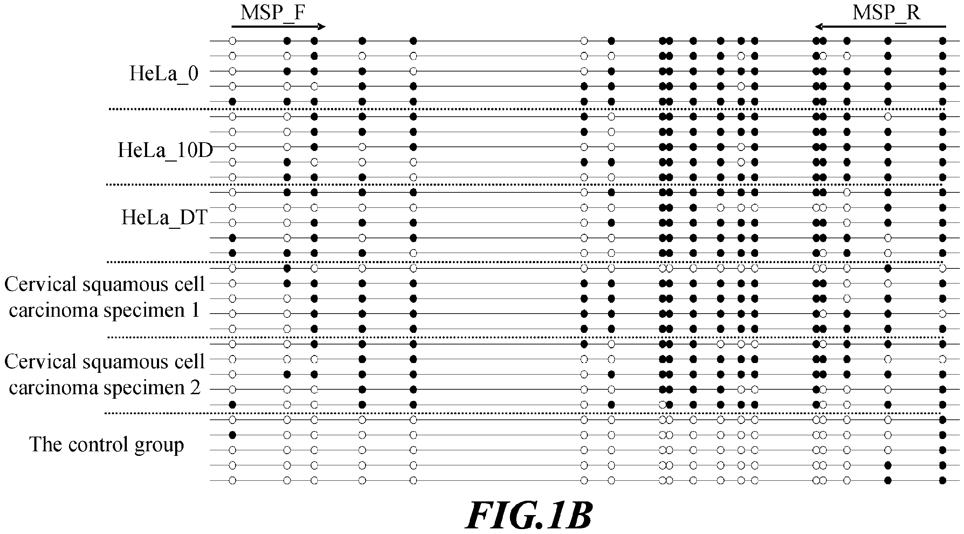

Figure 1B shows the result obtained by performing bisulfite sequencing (BS) analysis on various cervical specimens using a target gene ZNF582 in the inventive cancer screening method;

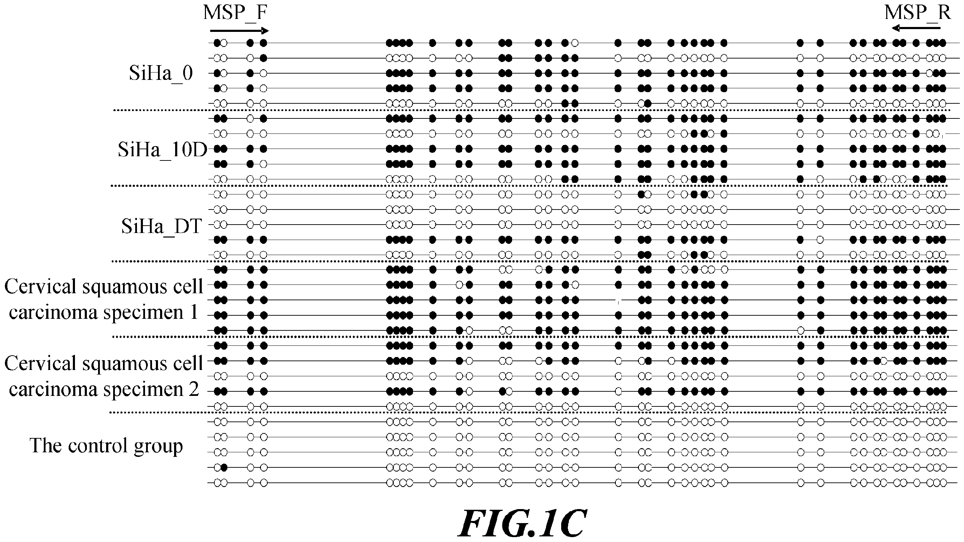

Figure 1C shows the result obtained by performing bisulfite sequencing (BS) analysis on various cervical specimens using a target gene PDE8B in the inventive cancer screening method; and

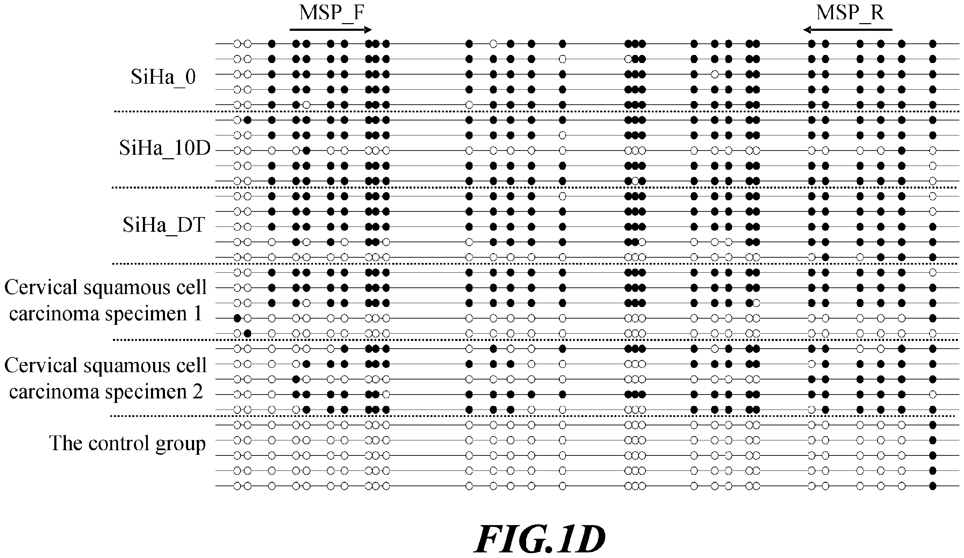

Figure 1D shows the result obtained by performing bisulfite sequencing (BS) analysis on various cervical specimens using a target gene DBC1 in the inventive cancer screening method.

### DETAILED DESCRIPTION OF THE PREFERRED EMBODIMENT

The invention will be illustrated more detailed with the following examples, but the invention is not limited thereto.

### Example 1 Materials and Methods

### 1. Materials

Test materials comprises a series of full cervical lesion specimens, including: squamous cell carcinoma (SCC, n = 20), adenocarcinoma (AC, n = 20), and normal cervical specimen (n = 10). All of these cervical specimen, ovarian specimen, and colon cancer specimen were obtained from Tri-service General Hospital, Taipei, ROC. Each specimen was subjected to genomic DNA extraction by means of QIAamp DNA Kit (QIAGEN), and was used in the comparison and analysis of DNA methylation condition within genome-wide DNA methylation. Prior to the analysis, the quality of genomic DNA was checked by Bioanalyzer (Agilent). In this example, 10µg of fragmented DNA was subjected to MeDIP (Methyl DNA IP) assay.

### 2. DNA methylation analysis by MeDIP and CpG island-Plus-Promoter arrays

Genomic DNA was fragmented to the size ranging from 300 to 1,000 bp by Bioruptor^{™} UCS-200 (Diagenode). We immunoprecipitated it for overnight at 4 °C with 30 µl of polyclonal Anti-5'-methyl cytosine antibody (Abcam) in a final volume of 100 µl IP buffer (0.15% SDS, 1% Triton X-100, 150mM NaCl, 1 mM EDTA, 0.5 mM EGTA, 10 mM Tris and 0.1% BSA). We incubated the mixture with 120 µl of Protein G Sepharose (Amersham) for 2 h at 4°C and washed it twice with 1 ml low salt, high salt buffer, lithium chloride and TE buffer. We then treated twice the protein G with elution buffer (1% SDS, 0.1M NaHCO₃) for 15 min at room temperature and recovered the Methylated DNA by phenol-chloroform extraction followed by ethanol precipitation. The enriched methylated DNA and input DNA were amplified by Whole Genome Amplification Kit (Sigma). Enriched and total DNA was end-labeled with Cy5 and Cy3, respectively, and co-hybridized to the CpG Island-Plus-Promoter Arrays designed and synthesized by NimbleGen Systems, Inc. This array contained 385,000 50-75 bp oligonucleotides tiled every 100 bp across the 24,659 HG18 RefSeq promoters (800 bp upstream to 200 bp down stream) and 28,226 CpG islands, present in duplicate.

Each feature on the array has a corresponding scaled log2-ratio. The log2-ratio is normalization to center the ratio data around zero. From the scaled log2-ratio data, a fixed-length window (500 bp) is placed around each consecutive probe and the one-sided Kolmogorov-Smirnov (KS) test is applied to determine whether the probes are drawn from a significantly more positive distribution than those in the rest of the array. The resulting score for each probe is the -log10 p-value from the windowed KS test around that probe (Scacheri et al, 2006). We detected enriched peaks by searching for at least 2 probes above a p-value minimum cutoff (-log10) of 2. Peaks within 300 bp of each other are merged. Differential methylation regions between SCC/AC and normal cervix within 2500 bp upperstream of transcription star sites were selected for future validation. Finally, p-value data was viewed using SignalMap (NimbleGen).

### 3. Bisulfite modification, methylation-specific polymerase chain reaction (MSP) and bisulfite sequencing (BS)

A DNA modification kit (Chemicon, Ternecula, CA) was used according to the manufacturer's recommendations to convert 1 µg aliquots of genomic DNA with sodium bisulfite to preserve the methylated cytosines. The final precipitate was eluted with 70 µl of pre-warmed (55°C) TE buffer for MSP.

Normal DNA of human peripheral blood was taken and subjected to bisulfite modification, which was used as the control group having un-methylation promoter sequence.

MSP was performed according to prior art. In short, 1 µl of modified DNA was amplified using MSP primers (table 1) that specifically recognized the methylated gene sequences present in the bisulfite-converted DNA. Methylation-specific PCR was done in a total volume of 25 µl containing 1 µl of modified template DNA, 1.5 pmol of each primer, 0.2 mmol/L deoxynucleotide triphosphates, and 1 unit of Gold *Taq* DNA polymerase (Applied Biosystems, Foster City, CA). MSP reactions were subjected to an initial incubation at 95°C for 5 min, followed by 35 cycles of 95°C for 30 s, and annealing at the appropriate temperature for 30 s and at 72°C for 30 s. The final extension was done at 72°C for 5 min. Amplification products were visualized on 2.5% agarose gel containing ethidium bromide and illuminated under UV light.

**Table 1. The sequences of MSP primers**

| Gene | | Primer | Sequence | |
|---|---|---|---|---|
| DBC1 | M | Forward (F') | 5' gttttcgtcgtttttcgttcggagatc 3' | (SEQ ID No: 5) |
| | | Reverse (R') | 5' gctctcgctctcgctattactcgct 3' | (SEQ ID No: 6) |
| PDE8B | M | Forward (F') | 5' tgtgtatgcgcgttttttcgttc 3' | (SEQ ID No: 7) |
| | | Reverse (R') | 5' acctatatatccgccgctccgtc 3' | (SEQ ID No: 8) |
| PTPRR | M | Forward (F') | 5' cggcgttgggtatgtttagtagtc 3' | (SEQ ID No: 9) |
| | | Reverse (R') | 5' aattacgaataaaaaaaacaaaaacgctc 3' | (SEQ ID No: 10) |
| ZNF582 | M | Forward (F') | 5' tgacggttttttgtttattcggttattc 3' | (SEQ ID No: 11) |
| | | Reverse (R') | 5'cgaacgcaaacgtacctacgc 3' | (SEQ ID No: 12) |

| | | | | |
|---|---|---|---|---|
| M: The primers can specifically recognize the methylated gene sequences present in the bisulfite-converted DNA. | | | | |

All MSP data were derived from at least two independent modifications of DNA. The absence of signal in duplicate experiments was scored as negative methylation. PCR product obtained by using MSP primer (M) that can recognize specifically methylation gene sequence was constructed into a pCR4-TOPO vector (Invitrogen, Carlsbad, CA). 5 independent clones were selected and were subjected to bisulfite sequencing (BS). Primers used in bisulfite sequencing (BS) were listed in Table 2. Bisulfite sequencing was carried out using 377 Auto-sequencer (Applied Biosystems, Foster City, CA). Sequenced results were shown in Sequence List. Sequence numbers in bisulfite sequencing were: DBC1_BS (SEQ ID No: 21), PDE8B_BS (SEQ ID No: 22), PTPRR_BS (SEQ ID No: 23) and ZNF582_BS (SEQ ID No: 24), respectively.

**Table 2. The sequences of bisulfite sequencing primers**

| Gene | Primer | Sequence | |
|---|---|---|---|
| DBC1 | Forward (F') | 5'ggttaagtttttttttyggygtagtt 3' | (SEQ ID No: 13) |
| | Reverse (R') | 5'tactccctctacctcccractctctc 3' | (SEQ ID No: 14) |
| PDE8B | Forward (F') | 5'ttgtggygtagaggattattagtttggt 3' | (SEQ ID No: 15) |
| | Reverse (R') | 5'ctaaaaacrcaacccatccctc 3' | (SEQ ID No: 16) |
| PTPRR | Forward (F') | 5'ggaattttattttgaaatttttttgtt 3' | (SEQ ID No: 17) |
| | Reverse (R') | 5'ccccacttcaaataaaatactattaaaaaaaac 3' | (SEQ ID No: 18) |
| ZNF582 | Forward (F') | 5'tagtgayggttttttgtttattyggttatt 3' | (SEQ ID No: 19) |
| | Reverse (R') | 5'taaacrtaaaaacaacaacccracct 3' | (SEQ ID No: 20) |

### Example 2 Screening of methylated target gene in cervical cancer

After screened by CpG island-Plus-Promoter arrays, four target genes that might have been highly methylated in the cervical cancer cell were screened, namely, DBC1 (SEQ ID No: 1), PDE8B (SEQ ID No: 2), PTPRR (SEQ ID No: 3) and ZNF582 (SEQ ID No: 4), respectively. Their detailed information was shown in Table 3. It could be seen from Table 3 that, among these four genes, except DBC1 that was known to be associated with bladder cancer, little study up to date had revealed the relation of those genes to cervical cancer.

**Table 3. Characteristics of methylated genes in cervical cancer that were identified using a CpG island-Plus-Promoter microarray.**

| Gene | UniGene | Chromosomal location | Full name | SEQ ID No |
|---|---|---|---|---|
| DBC1 | NM_014618 | 9q32-q33 | deleted in bladder cancer 1 | SEQ ID No: 1 |
| PDE8B | NM_003719 | 5q14.1 | phosphodiesterase 8B | SEQ ID No: 2 |
| PTPRR | NM_002849 | 12q15 | protein tyrosine phosphatase, receptor type, R | SEQ ID No: 3 |
| ZNF582 | NM_144690 | 19q13.43 | zinc finger protein 582 | SEQ ID No: 4 |

### Example 3 The analysis of methylation state of target gene in cervical lesion specimen by bisulfite sequencing (BS)

### The target gene : PTPRR

Test specimen groups:
1. HeLa_0: HeLa cervical cancer cell line;
2. HeLa_10D: HeLa cervical cancer cell line (HeLa_0) treated with 10 µM DNA methyltransferase inhibitor 5'-aza-2'-deoxycytidine (AZC);
3. HeLa_DT: HeLa cervical cancer cell line (HeLa_0) treated with both of 10 µM DNA methyltransferase inhibitor 5'-aza-2'-deoxycytidine (AZC) and 0.33 µM TSA (Sigma Chemical Co., St. Louis, MO);
4. AC: Cervical adenocarcinoma specimen;
5. SCC: Cervical squamous cell carcinoma specimen;
6. Control group (normal): normal cervical blood DNA as the control group without methylation.

Each of those test specimens described above was subjected to bisulfite modification, and then bisulfite sequencing (BS) to analyze whether the target gene (PTPRR) in each test specimen has been hypermethylated or not. The result was shown in Fig. 1, where black indicated methylation region, and white indicated un-methylation region. The target gene PTPRR had not been methylated in the control group and the adenocarcinoma, while the target gene PTPRR had been hypermethylated in HeLa cervical cancer cell line (HeLa_0) and cervical squamous cell carcinoma specimen (SCC). Consequently, the methylation rate of PTPRR could be used as biomarker to screen whether there was cervical cancer or not.

In addition, in order to ascertain whether methylation rate of the target gene in cervical cancer specimen could be regulated through DNA methylation, HeLa cervical cancer cell line was treated with 10 µM DNA methyltransferase inhibitor 5'-aza-2'-deoxycytidine (AZC) (Sigma Chemical Co.). The DNA specimen extracted from said cell was subjected to bisulfite modification and then bisulfite sequencing (BS). Result shown in Fig. 1A indicated that, compared with the cervical squamous cell carcinoma specimen (SCC) and HeLa cervical cancer cell line (HeLa_0), the target gene PTPRR in the AZC-treated HeLa cervical cancer cell line (HeLa_10D) had part of region been de-methylated.

Trichostatin A (TSA) is histone deacetylase (HDAC) inhibitors, and can be used to lower or degrade methylation rate. HeLa cervical cancer cell line (HeLa_DT) has been treated with both AZC and TSA. The result shown in Fig. 1 indicated that, compared with cervical squamous cell carcinoma specimen (SCC) and HeLa cervical cancer cell line (HeLa_0), the target gene PTPRR in the AZC- and TSA-treated HeLa cervical cancer cell line (HeLa_DT) had been highly de-methylated.

In summary, the above-described results had confirmed that the target gene PTPRR in cervical cancer specimen had been methylated through DNA methylation.

### The target gene : ZNF582

Test specimen groups:
1. HeLa_0: HeLa cervical cancer cell line;
2. HeLa_10D: the HeLa cervical cancer cell line (HeLa_0) treated with 10 µM DNA methyltransferase inhibitor 5'-aza-2'-deoxycytidine (AZC);
3. HeLa_DT: the HeLa cervical cancer cell line (HeLa_0) treated with both of 10 µM DNA methyltransferase inhibitor 5'-aza-2'-deoxycytidine (AZC) and 0.33 µM TSA;
4. SCC 1: Cervical squamous cell carcinoma specimen 1;
5. SCC 2: Cervical squamous cell carcinoma specimen 2;
6. The control group (normal): normal cervical blood DNA as the un-methylation control group;

Wherein cervical squamous cell carcinoma specimen 1 and specimen 2 were specimens obtained from different patients.

Those test specimens mentioned above were subjected to bisulfite sequencing (BS) to analyze whether the target gene (ZNF582) in each test specimen had been hypermethylated or not. The result shown in Fig. 1B indicated that, compared with the control group, the target gene ZNF582 in cervical squamous cell carcinoma specimen 1 (SCC), cervical squamous cell carcinoma specimen 2 (SCC) and HeLa cervical cancer cell line (HeLa_0) had been hypermethylated. Consequently, the target gene ZNF582 in cervical cancer specimens would be highly methylated.

### The target gene : PDE8B

Test specimen groups:
1. SiHa_0: SiHa cervical cancer cell line;
2. SiHa_10D: SiHa cervical cancer cell line (SiHa_0) treated with 10 µM DNA methyltransferase inhibitor 5'-aza-2'-deoxycytidine (AZC);
3. SiHa_DT: SiHa cervical cancer cell line (SiHa_0) treated with both of 10 µM DNA methyltransferase inhibitor 5'-aza-2'-deoxycytidine(AZC) and 0.33 µM TSA;
4. SCC 1: Cervical squamous cell carcinoma specimen 1;
5. SCC 2: Cervical squamous cell carcinoma specimen 2;
6. The control group (normal): normal cervical blood DNA used as un-methylation control group;
wherein cervical squamous cell carcinoma specimen 1 and specimen 2 were specimens obtained from different patients.

Those test specimens described above were subjected to bisulfite sequencing (BS) to analyze whether the target gene (PDE8B) in each test specimen had been hypermethylated or not. The result shown in Fig. 1C indicated that, compared with the control group, the target gene PDE8B in cervical squamous cell carcinoma specimen 1 (SCC 1), cervical squamous cell carcinoma specimen 2 (SCC 2) and SiHa cervical cancer cell line (SiHa_0) had been hypermethylated. Consequently, the target gene PDE8B in cervical cancer specimens would be highly methylated.

### The target gene : DBC1

Test specimen groups:
1. SiHa_0: SiHa cervical cancer cell line;
2. SiHa_10D: SiHa cervical cancer cell line (SiHa_0) treated with 10 µM DNA methyltransferase inhibitor 5'-aza-2'-deoxycytidine (AZC);
3. SiHa_DT: SiHa cervical cancer cell line (SiHa_0) treated with both of 10 µM DNA methyltransferase inhibitor 5'-aza-2'-deoxycytidine (AZC) and 0.33 µM TSA;
4. SCC 1: Cervical squamous cell carcinoma specimen 1;
5. SCC 2: Cervical squamous cell carcinoma specimen 2;
6. The control group (normal): normal cervical blood DNA as the un-methylation control group;
wherein cervical squamous cell carcinoma specimen 1 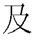 specimen 2 were specimens obtained from different patients.

Those test specimens described above were subjected to bisulfite sequencing (BS) to analyze whether the target gene (DBC1) in each test specimen had been hypermethylated. The result shown in Fig. ID indicated that, compared with the control group, the target gene DBC1 in cervical squamous cell carcinoma specimen 1 (SCC 1), cervical squamous cell carcinoma specimen 2 (SCC 2) and SiHa cervical cancer cell line (SiHa_0) had been hypermethylated. Consequently, the target gene DBC1 in cervical cancer specimens would be highly methylated.

### Example 4 Methylation analysis of the target gene in cervical cancer specimens

Methylation-specific PCR (MSP) was used to analyze the methylation state of said four target genes in cervical squamous cell carcinoma (SCC) specimens. The methylation state analysis result shown in Table 4 indicated that, in normal cervical specimen, methylation frequency of DBC1, PDE8B, PTPRR and ZNF582 was 11%, 0%, 9% and 6%, respectively; in cervical squamous cell carcinoma specimen, the methylation frequency of DBC1, PDE8B, PTPRR and ZNF582 was 100%, 47%, 100% and 97%, respectively. It could be known from these results that, in cervical squamous cell carcinoma specimens, said four genes were highly methylated. Consequently, methylation rate of DBC1, PDE8B, PTPRR and ZNF582 could be used indeed as the screening index in screening cervical cancer.

**Table 4 Methylation state analysis of the target gene in cervical squamous cell carcinoma specimens**

| | Target Genes CGI methylation rate (%) | |
|---|---|---|
| | Normal (n=54) | SCC tissue (n=30) |
| DBC1 | 11% | 100% |
| PDE8B | 0% | 47% |
| PTPRR | 9% | 100% |
| ZNF582 | 6% | 97% |

### Example 5 Methylation analysis of the target gene in ovarian tumor specimen

Methylation-specific PCR (MSP) was used to analyze the methylation state of the target gene in ovarian tumor specimens. Methylation state analysis results in Table 5 reported the analysis of methylation states of three genes DBC1, PTPRR and ZNF582 in ovarian malignant tumor specimen and ovarian benign tumor specimen. The results indicated that, the methylation frequency of DBC1, PTPRR and ZNF582 in ovarian malignant tumor specimen was 50.3%, 50.0% and 56.3%, respectively; the methylation frequency of DBC1, PTPRR and ZNF582 in ovarian benign tumor specimen was 2.5%, 0.0% and 12.5%, respectively. The differential methylation level was 53.8%, 50.0% and 43.8%, respectively. Consequently, compared with ovarian benign tumor specimen, methylation levels of said three genes in ovarian malignant tumor specimen were remarkably higher. Therefore, methylation rate of DBC1, PTPRR and ZNF582 could be used indeed as the screening index in screening ovarian cancer.

**Table 5 Methylation state analysis of the target gene in ovarian tumor specimens**

| | Malignance_Ov (n=28) | Benign_Ov (n=28) | Differential Methylation_level |
|---|---|---|---|
| DBC1 | 56.3% | 2.5% | 53.8% |
| PTPRR | 50.0% | 0.0% | 50.0% |
| ZNF582 | 56.3% | 12.5% | 43.8% |

### Example 6 Methylation analysis of the target gene in colon cancer specimen

Methylation-specific PCR (MSP) was used to analyze the methylation state of the target gene in colon cancer specimen. The methylation state analysis result in Table 6 reported the methylation state of four genes, DBC1, PDE8B, PTPRR and ZNF582, in colon cancer specimen. The result indicated that, methylation frequencies of DBC1, PDE8B, PTPRR and ZNF582 in colon cancer specimen was 100.0%, 100.0, 100.0% and 100.0%, respectively; while methylation frequencies of DBC1, PDE8B, PTPRR and ZNF582 in normal colon tissue specimen were 25.0%, 25.0%, 25.0% and 25.0%, respectively. Consequently, compared with normal colon tissue specimen, methylation levels of said four genes in colon cancer specimen were remarkably higher. Therefore, methylation rates of DBC1, PDE8B, PTPRR and ZNF582 could be used indeed as the screening index in screening colon cancer.

**Table 6 Methylation state analysis of the target gene in colon cancer specimen**

| | Target Genes CGI methylation rate (%) | |
|---|---|---|
| | Normal colon tissue specimen (n=24) | Colon cancer specimen (n=20) |
| DBC1 | 25% | 100% |
| PDE8B | 25% | 100% |
| PTPRR | 25% | 100% |
| ZNF582 | 25% | 100% |

The cancer screening method provided by the invention has following advantages over the above-described conventional techniques:
1. The cancer screening method, biomarker and use therof, provided by the invention uses the methylation rate of a particular gene in a isolated specimen as the biomarker to determine the risk of having cancer, and as compared with conventional methods such as cervical scraping and human papilloma virus testing (HPV testing), both of sensitivity and specificity of the inventive cancer screening method are higher than those of the two above-described methods.
2. The cancer screening method, biomarker and use therof, provided by the invention can be used not only as the first line cervical cancer screening, but also in combination with or assisting human papilloma virus testing (HPV testing) as the second line cervical cancer screening, so as to achieve more accurate cervical cancer screening effect.
3. The cancer screening method, biomarker and use therof, provided by the invention can be applied on not only the screening of cervical cancer, but also on the screening of other cancer (for example: ovarian cancer, colon cancer and the like) to assist the diagnosis of abnormal specimen.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A cancer screening method, based on detecting the methylation rate of a target gene in a test specimen, and using the methylation rate as the screening index to determine the risk of having cancer, the method comprising following steps:
step 1: providing a test specimen;
step 2: detecting the methylation rate of a CpG sequence in at least one target gene of said test specimen, wherein the target gene comprises at least one selected from the group consisting of DBC1, PTPRR and ZNF582; and
step 3: determining whether there is cancer or not in the specimen based on the methylation rate in the target gene,
wherein the cancer is selected from the group consisting of cervical cancer, colon cancer and ovarian cancer.

2. A cancer screening method as recited in claim 1, wherein the test specimen is an isolated specimen selected from the group consisting of full cervical lesion specimens, including squamous cell carcinoma and adenocarcinoma.

3. A cancer screening method as recited in claim 1, wherein the testing method for the methylation state of CpG sequence of the target gene comprising methylation-specific polymerase chain reaction (MSP), quantitative methylation-specific polymerase chain reaction (QMSP), bisulfite sequencing (BS), microarrays, mass spectrometry, denaturing high-performance liquid chromatography (DHPLC), and pyrosequencing.

4. A cancer screening method as recited in claim 1, wherein the target gene DBC1 has the nucleotide sequence as depicted in SEQ ID No: 1, the target gene PTPRR has the nucleotide sequence as depicted in SEQ ID No: 3, and the target gene ZNF582 has the nucleotide sequence as depicted in SEQ ID No: 4.

5. A cancer screen method as recited in claim 4, wherein the methylated bisulfite sequencing of the target gene DBC1 is recognized by primers as depicted in SEQ ID No: 13, 14, the target gene PTPRR has the nucleotide sequence as depicted in SEQ ID No: 17, 18, and the target gene ZNF582 has the nucleotide sequence as depicted in SEQ ID No: 19, 20.

6. A cancer screen method as recited in claim 1, wherein the target gene DBC1 is recognized by primers as depicted in SEQ ID No: 5, 6, the target gene PTPRR has the nucleotide sequence as depicted in SEQ ID No: 9, 10, and the target gene ZNF582 has the nucleotide sequence as depicted in SEQ ID No: 11, 12.

7. A cancer screening method as recited in claim 1, wherein in the case of cervical cancer the test specimen is the HPV(human papilloma virus)-positive cervical cell specimen.

8. A cancer screening method as recited in claim 1, wherein in the case of ovarian cancer the test specimen is ovarian cancer tissue.

## Patentansprüche

1. Krebs-Screening-Verfahren, basierend auf der Erfassung der Methylierungsrate eines Zielgens in einer Probe, und unter Verwendung der Methylierungsrate als Screening-Index, um das Risiko einer Krebserkrankung zu bestimmen, wobei das Verfahren folgende Phasen umfasst:
Phase 1: Bereitstellung einer Probe;
Phase 2: Erfassung der Methylierungsrate einer CpG-Sequenz in mindestens einem Zielgen der Probe, wobei das Zielgen mindestens eines aus folgender Gruppe umfasst: DBC1, PTPRR und ZNF582; und
Phase 3: Bestimmung, ob in der Probe Krebs vorliegt oder nicht, auf der Grundlage der Methylierungsrate in dem Zielgen,
wobei der Krebs aus folgender Gruppe ausgewählt ist: Gebärmutterhalskrebs, Darmkrebs und Eierstockkrebs.

2. Krebs-Screening-Verfahren nach Anspruch 1, wobei die Probe eine isolierte Probe ausgewählt aus folgender Gruppe ist: vollen Proben zervikaler Läsion, einschließlich Plattenepithelkarzinom und Adenokarzinom.

3. Krebs-Screening-Verfahren nach Anspruch 1, wobei das Testverfahren für den Methylierungszustand der CpG-Sequenz des Zielgens methylierungsspezifische Polymerasenkettenreaktion (MSP) umfasst, quantitative methylierungsspezifische Polymerasenkettenreaktion (QMSP), Bisulfit-Sequenzierung (BS), Microarrays, Massenspektrometrie, denaturierende Hochleistungs-Flüssigkeitschromatografie (DHPLC) und Pyrosequenzierung.

4. Krebs-Screening-Verfahren nach Anspruch 1, wobei das Zielgen DBC1 die Nukleotidsequenz hat wie in SEQ ID ID No.: 1 dargestellt, das Zielgen PTPRR hat die Nukleotidsequenz wie in SEQ ID ID No.: 3 dargestellt, und das Zielgen ZNF582 hat die Nukleotidsequenz wie in SEQ ID ID No.: 4 dargestellt.

5. Krebs-Screening-Verfahren nach Anspruch 4, wobei die methylierte Bisulfit-Sequenzierung des Zielgens DBC1 von Primern erkannt wird wie in SEQ ID ID No.: 13, 14 dargestellt, das Zielgen PTPRR hat die Nukleotidsequenz wie in SEQ ID ID No.: 17, 18 dargestellt, und das Zielgen ZNF582 hat die Nukleotidsequenz wie in SEQ ID ID No.: 19, 20 dargestellt.

6. Krebs-Screening-Verfahren nach Anspruch 1, wobei das Zielgen DBC1 von Primern erkannt wird wie in SEQ ID ID No.: 5, 6 dargestellt, das Zielgen PTPRR hat die Nukleotidsequenz wie in SEQ ID ID No.: 9, 10 dargestellt, und das Zielgen ZNF582 hat die Nukleotidsequenz wie in SEQ ID ID No.: 11, 12 dargestellt.

7. Krebs-Screening-Verfahren nach Anspruch 1, wobei im Falle von Gebärmutterhalskrebs die Probe die HPV(humanes Papillomavirus)-positive Gebärmutterhalszellenprobe ist.

8. Krebs-Screening-Verfahren nach Anspruch 1, wobei im Falle von Eierstockkrebs die Probe Eierstockkrebsgewebe ist.

## Revendications

1. Procédé de dépistage du cancer, basé sur la détection du taux de méthylation d'un gène cible dans un échantillon d'essai, et l'utilisation du taux de méthylation comme indice de dépistage pour déterminer le risque d'avoir un cancer, le procédé comprenant les étapes suivantes :
étape 1 : se procurer un échantillon d'essai ;
étape 2 : détecter le taux de méthylation d'une séquence CpG dans au moins un gène cible dudit échantillon d'essai, le gène cible comprenant au moins l'un choisi dans le groupe consistant en DBC1, PTPRR et ZNF582 ; et
étape 3 : déterminer s'il y a un cancer ou non dans l'échantillon sur la base du taux de méthylation dans le gène cible,
le cancer étant choisi dans le groupe consistant en le cancer du col de l'utérus, le cancer du côlon et le cancer de l'ovaire.

2. Procédé de dépistage du cancer selon la revendication 1, dans lequel l'échantillon d'essai est un échantillon isolé choisi dans le groupe consistant en des échantillons globaux de lésion du col de l'utérus, comprenant le carcinome squameux et l'adénocarcinome.

3. Procédé de dépistage du cancer selon la revendication 1, dans lequel le procédé de test pour l'état de méthylation de la séquence CpG du gène cible comprenant la réaction en chaîne de la polymérase spécifique de la méthylation (MSP), la réaction en chaîne de la polymérase spécifique de la méthylation quantitative (QMSP), le séquençage au bisulfite (BS), les microréseaux, la spectrométrie de masse, la chromatographie liquide haute performance dénaturante (DHPLC) et le pyroséquençage.

4. Procédé de dépistage du cancer selon la revendication 1, dans lequel le gène cible DBC1 a la séquence nucléotidique telle que représentée dans SEQ ID ID NO : 1, le gène cible PTPRR a la séquence nucléotidique telle que représentée dans SEQ ID ID NO : 3, et le gène cible ZNF582 a la séquence nucléotidique telle que représentée dans SEQ ID ID NO : 4.

5. Procédé de dépistage du cancer selon la revendication 4, dans lequel le séquençage au bisulfite méthylé du gène cible DBC1 est reconnu par des amorces telles que représentées dans SEQ ID ID NO : 13, 14, le gène cible PTPRR a la séquence nucléotidique telle que représentée dans SEQ ID ID NO : 17, 18, et le gène cible ZNF582 a la séquence nucléotidique telle que représentée dans SEQ ID ID NO : 19, 20.

6. Procédé de dépistage du cancer selon la revendication 1, dans lequel le gène cible DBC1 est reconnu par des amorces telles que représentées dans SEQ ID ID NO : 5, 6, le gène cible PTPRR a la séquence nucléotidique telle que représentée dans SEQ ID ID NO : 9, 10, et le gène cible ZNF582 a la séquence nucléotidique telle que représentée dans SEQ ID ID NO : 11, 12.

7. Procédé de dépistage du cancer selon la revendication 1, dans lequel, dans le cas du cancer du col de l'utérus, l'échantillon d'essai est l'échantillon de cellules de col de l'utérus HPV (virus du papillome humain) positif.

8. Procédé de dépistage du cancer selon la revendication 1, dans lequel, dans le cas du cancer de l'ovaire, l'échantillon d'essai est un tissu de cancer de l'ovaire.
